(19) Europäisches Patentamt / European Patent Office / Office européen des brevets

(11) **EP 1 594 401 B1**

(12) **EUROPEAN PATENT SPECIFICATION**

(45) Date of publication and mention
of the grant of the patent:
**22.08.2018 Bulletin 2018/34**

(21) Application number: **03710727.3**

(22) Date of filing: **15.01.2003**

(51) Int Cl.:
*A61B 5/05* (2006.01)    *A61B 5/0488* (2006.01)
*A61B 5/00* (2006.01)    *A61B 17/00* (2006.01)

(86) International application number:
**PCT/US2003/002056**

(87) International publication number:
**WO 2004/064634 (05.08.2004 Gazette 2004/32)**

(54) **SYSTEMS FOR DETERMINING DIRECTION TO A NERVE**

SYSTEME ZUR BESTIMMUNG DER RICHTUNG ZU EINEM NERV

SYSTEMES PERMETTANT DE DETERMINER UNE DIRECTION VERS UN NERF

(84) Designated Contracting States:
**DE FR GB**

(43) Date of publication of application:
**16.11.2005 Bulletin 2005/46**

(73) Proprietors:
• **NuVasive, Inc.**
**San Diego CA 92121 (US)**
• **Blewett, Jeannette**
**Helena, MT 59601 (US)**

(72) Inventors:
• **FARQUHAR, Allen**
**San Diego, CA 92117 (US)**
• **GHARIB, James**
**San Diego, CA 92130 (US)**

• **KAULA, Norbert**
**Arvada, CO 80005 (US)**
• **BLEWLETT, Jeffrey DI**
**deceased (US)**

(74) Representative: **Boult Wade Tennant LLP**
**Verulam Gardens**
**70 Gray's Inn Road**
**London WC1X 8BT (GB)**

(56) References cited:
**WO-A1-00/38574        WO-A1-01/37728**
**WO-A2-03/026482      US-A- 5 284 154**
**US-A- 5 409 652        US-A- 5 775 331**
**US-B1- 6 466 817      US-B1- 6 564 078**

**Description**

**BACKGROUND**

**I. Field**

The present application relates to a system and methods generally aimed at surgery. More particularly, the present application relates to a system and related methods for determining the direction of a surgical instrument to a nerve during surgical access procedures.

**II. Description of Related Art**

A variety of surgeries involve establishing a working channel to gain access to a surgical target site. Oftentimes, based on the anatomical location of the surgical target site (as well as the approach thereto), the instruments required to form or create or maintain the working channel may have to pass near or close to nerve structures which, if contacted or disturbed, may be problematic to the patient. Examples of such "nerve sensitive" procedures may include, but are not necessarily limited to, spine surgery and prostrate or urology-related surgery.

Systems and methods exist for monitoring nerves and nerve muscles. One such system determines when a needle is approaching a nerve. The system applies a current to the needle to evoke a muscular response. The muscular response is visually monitored, typically as a shake or "twitch". When such a muscular response is observed by the user, the needle is considered to be near the nerve coupled to the responsive muscle. These systems require the user to observe the muscular response (to determine that the needle has approached the nerve). This may be difficult depending on the competing tasks of the user. In addition, when general anaesthesia is used during a procedure, muscular response may be suppressed, limiting the ability of a user to detect the response.

While generally effective (although crude) in determining nerve proximity, such existing systems are incapable of determining the direction of the nerve to the needle or instrument passing through tissue or passing by the nerves. While the surgeon may appreciate that a nerve is in the general proximity of the instrument, the inability to determine the direction of the nerve relative to the instrument can lead to guess work by the surgeon in advancing the instrument, which raises the spectre of inadvertent contact with, and possible damage to, the nerve.

WO 2003/026482, which falls under Article 54(3) EPC, describes a system for neuro-physiology-based monitoring to determine nerve proximity and nerve direction. A surgical accessory is equipped with stimulation electrodes which provide stimulation current signals. The response of a nerve to stimulation current signals is monitored and a threshold stimulation current for each electrode is determined using a bracketing and bisection method. From this information, the angular direction from a stimulation electrode to a nerve is determined and displayed to a user, for example, as an arrow.

**SUMMARY**

The present application may be directed to at least reduce the effects of the above-described problems with the prior art. The present disclosure describes a system and related methods for determining the direction of a surgical instrument to a nerve during surgical procedures. According to one aspect of the system, this involves the use of neurophysiology-based monitoring to determine nerve direction to surgical instruments employed in accessing a surgical target site. The system may do so in an automated, easy to use, and easy to interpret fashion so as to provide a surgeon-driven system.

The system may combine neurophysiology monitoring with any of a variety of instruments used in or in accessing a surgical target site (referred to herein "surgical access instruments"). By way of example only, such surgical access instruments may include, but are not necessarily limited to, any number of devices or components for creating an operative corridor to a surgical target site, such as K-wires, sequentially dilating cannula systems, distractor systems, and/or retractor systems.

A general method described in the present disclosure may include: (a) providing multiple (e.g., four orthogonally-disposed) electrodes around the periphery of the surgical access instrument; (b) stimulating the electrodes to identify the current threshold ($I_{Thresh}$) necessary to innervate the muscle myotome coupled to the nerve near the surgical access instrument; (c) determining the direction of the nerve relative to the surgical access instrument via successive approximation; and (d) communicating this successive approximation direction information to the surgeon in an easy-to-interpret fashion.

The act of providing multiple (e.g., four orthogonally-disposed) electrodes around the periphery of the surgical access instrument may be accomplished in any number of suitable fashions depending upon the surgical access instrument in question. For example, the electrodes may be disposed orthogonally on any or all components of a sequential dilation system (including an initial dilator, dilating cannulae, and working cannula), as well as speculum-type and/or retractor-based access systems. The act of stimulating is accomplished by applying any of a variety of suitable stimulation signals to the electrode(s) on the surgical accessory, including current pulses of varying magnitude and/or frequency. The stimulating act may be performed during and/or after the process of creating an operative corridor to the surgical target site.

The act of determining the direction of the surgical access instrument relative to the nerve via successive approximation is preferably performed by monitoring or measuring the EMG responses of muscle groups associated with a particular nerve and innervated by the nerve(s) stimulated during the process of gaining surgical access to a desired surgical target site.

The act of communicating this successive approximation information to the surgeon in an easy-to-interpret fashion may be accomplished in any number of suitable fashions, including but not limited to the use of visual indicia (such as alpha-numeric characters, light-emitting elements, and/or graphics) and audio communications (such as a speaker element). By way of example only, this may include providing an arc or other graphical representation that indicates the general direction to the nerve. The direction indicator may quickly start off relatively wide, become successively more narrow (based on improved accuracy over time), and may conclude with a single arrow designating the relative direction to the nerve.

Communicating this successive approximation information may be an important feature. By providing such direction information, a user will be kept informed as to whether a nerve is too close to a given surgical accessory element during and/or after the operative corridor is established to the surgical target site. This is particularly advantageous during the process of accessing the surgical target site in that it allows the user to actively avoid nerves and redirect the surgical access components to successfully create the operative corridor without impinging or otherwise compromising the nerves.

Based on this nerve direction feature, an instrument is capable of passing through virtually any tissue with minimal (if any) risk of impinging or otherwise damaging associated neural structures within the tissue, thereby making the system suitable for a wide variety of surgical applications.

A direction-finding algorithm that finds a stimulation threshold current one electrode at a time for a plurality of electrodes (e.g., four electrodes) may require 40 to 80 stimulations in order to conclude with a direction vector. At a stimulation rate of 10 Hz, this method may take four to eight seconds before any direction information is available to a surgeon. A surgeon may grow impatient with the system. An "arc" method described herein may improve the direction-finding algorithm and provide nerve direction information to the surgeon sooner. The system may display direction to the nerve during a sequence of stimulations as an "arc" (or wedge), which represents a zone containing the nerve. Computation of the direction arc (wedge) may be based on stimulation current threshold ranges, instead of precise, finally-calculated stimulation current threshold levels. Display of the direction arc (wedge) is possible at any time that the stimulation current thresholds are known to fall within a range of values.

The present invention is a system as defined in the independent claim 1. Preferred embodiments are set forth in the dependent claims.

The surgical accessory of the claimed system comprises a system for establishing an operative corridor to a surgical target site. The system for establishing an operative corridor may comprise a series of sequential dilation cannulae, where at least one cannula has said plurality of stimulation electrodes near a distal end. The system for establishing an operative corridor may further comprise a K-wire. The K-wire may have a first stimulation electrode at a distal tip. The K-wire may have a second stimulation electrode away from the distal tip. The K-wire may be slidably received in the surgical accessory. The system for establishing an operative corridor is configured to access a spinal target site. The system for establishing an operative corridor is configured to establish an operative corridor via a lateral, trans-psoas approach.

The system may further comprise a handle coupled to the surgical accessory. The handle may have at least one button for initiating the electrical stimulation from the control unit to a plurality of stimulation electrodes on the surgical accessory.

The control unit may comprise a display operable to display an electromyographic (EMG) response of the muscle. The control unit may comprise a touch-screen display operable to receive commands from a user.

The stimulation electrodes may be positioned near a distal end of the surgical accessory. The stimulation electrodes may be positioned in a two-dimensional plane. The stimulation electrodes may be positioned orthogonally to form a cross. The control unit may derive x and y Cartesian coordinates of a nerve direction with respect to the surgical accessory by using $x = i_w^2 - i_e^2$ and $y = i_s^2 - i_n^2$ where $i_e$, $i_w$, $i_n$, and $i_s$ represent stimulation current thresholds for east, west, north, and south electrodes. The stimulation electrodes may comprise a first set of electrodes in a first two-dimensional plane and a second set of at least one electrode in another plane that is parallel to the first plane. The stimulation electrodes may form a tetrahedron. The control unit may be configured to determine a three-dimensional vector from a reference point on the surgical accessory to a nerve.

The stimulation electrodes may comprise two pairs of electrodes. The stimulation electrodes may comprise a first electrode at a first longitudinal level of the surgical accessory and a second electrode at a second longitudinal level of the surgical accessory.

The control unit is configured to electrically stimulate a first stimulation electrode with a first current signal, determine whether a first stimulation current threshold has been bracketed, stimulate a second stimulation electrode with a second current signal, and determine whether a second stimulation current threshold has been bracketed. The first and second current signals may be equal. The control unit is further configured to determine a first range for the first stimulation current threshold, and determine a second range for the second stimulation current threshold. Each range has a maximum stimulation current threshold value and a minimum stimulation current threshold value.

The control unit is configured to process the first and second ranges and display an arc indicating a general direction of a nerve from the surgical accessory. The control unit is further configured to electrically stimulate the first stimulation electrode with a third current signal, determine whether the first stimulation current threshold has been bracketed, stimulate the second stimulation electrode with a fourth current signal, and determine whether the second stimulation current threshold has been bracketed.

The control unit is configured to electrically stimulate each electrode until a stimulation current threshold has been bracketed. The control unit is configured to display an arc indicating a general direction of a nerve from the surgical accessory and narrow the arc as stimulation current thresholds are bracketed. The control unit is further configured to electrically stimulate the first and second stimulation electrodes to bisect each bracket until a first stimulation current threshold has been found for the first stimulation electrode and a second stimulation current threshold has been found for the second stimulation electrode within a predetermined range of accuracy. The control unit configured to display an arc indicating a general direction of a nerve from the surgical accessory and narrow the arc as stimulation current threshold brackets are bisected.

The control unit may be configured to emit a sound when the control unit determines a distance between the surgical accessory and the nerve has reached a predetermined level. The control unit may be configured to emit a sound that indicates a distance between the surgical accessory and the nerve. The surgical accessory may be dimensioned to be inserted percutaneously through a hole to a surgical site.

**BRIEF DESCRIPTION OF THE DRAWINGS**

FIG. 1 is a flow chart illustrating fundamental functions of a neurophysiology-based surgical system according to one embodiment of the present application;

FIG. 2 is a perspective view of an exemplary surgical system capable of performing the functions in FIG. 1 and determining nerve direction to surgical instruments employed in accessing a surgical target site;

FIG. 3 is a block diagram of the surgical system shown in FIG. 2;

FIG. 4 is a graph illustrating a plot of a stimulation current pulse capable of producing a neuromuscular response (EMG) of the type shown in FIG. 5;

FIG. 5 is a graph illustrating a plot of the neuromuscular response (EMG) of a given myotome over time based on a current stimulation pulse (such as shown in FIG. 4) applied to a nerve bundle coupled to the given myotome;

FIG. 6 is a graph illustrating a plot of EMG response peak-to-peak voltage (Vpp) for each given stimulation current level ($I_{Stim}$) forming a stimulation current pulse (otherwise known as a "recruitment curve") for the system of FIG. 2;

FIG. 7A-7E are graphs illustrating a current threshold-hunting algorithm that may be used by the system of FIG. 2;

FIG. 8 illustrates four orthogonal electrodes near a distal end of a surgical instrument, such as a cannula, modeled as north, south, east and west points in a two-dimensional X-Y plane for the system of FIG. 2;;

FIG. 9 illustrates a nerve point (x, y) bounded by maximum and minimum x- and y-values, which forms a rectangle;

FIG. 10 illustrates a vector from an origin (center axis of an instrument with electrodes) to a nerve point (x, y) and an arc containing that vector found by the system of FIG. 2;

FIG. 11 illustrates a stimulation site and multiple EMG response sensing sites for the system of FIG. 2;

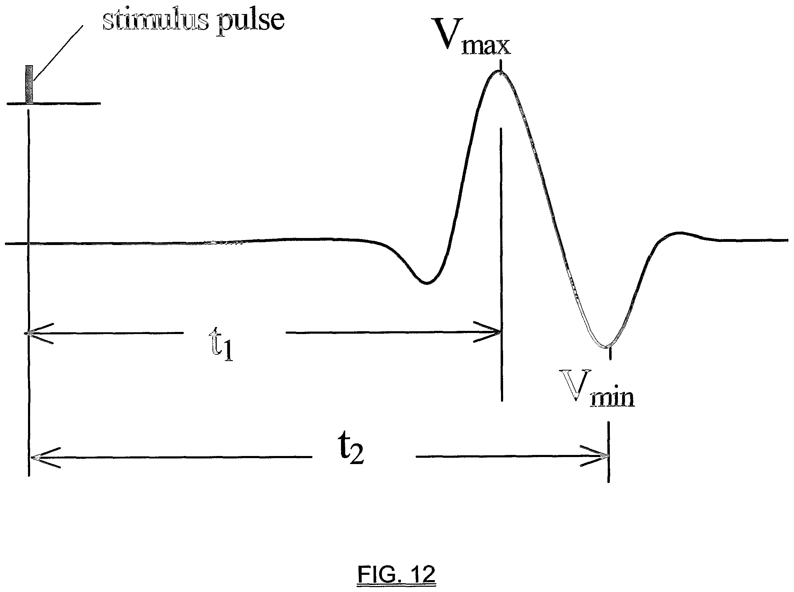

FIG. 12 is a graph illustrating a plot of a neuromuscular response (EMG) over time in response to a stimulus current pulse, where the plot shows voltage extrema at times T1 and T2;

FIG. 13 is a graph illustrating a method of determining the direction of a nerve (denoted as a "hexagon") relative to an instrument having four (4) orthogonally disposed stimulation electrodes (denoted by the "circles") for the system of FIG. 2;

FIG. 14A is a side view and FIG. 14B is a front view of a distal end of a surgical instrument, such as a cannula in FIG. 2, with four orthogonal electrodes and a fifth electrode;

FIG. 15A-15C are displays of a surgical instrument in FIG. 2 and a nerve direction arc that may become progressively smaller until it becomes an arrow as stimulation threshold levels are bracketed, bisected and found for a plurality of electrodes in FIGS. 14A-14B;

FIGS. 16-19 illustrate a sequential dilation access system of FIG. 2 in use creating an operative corridor to an intervertebral disk;

FIGS. 20-21 are exemplary screen displays illustrating one embodiment of the nerve direction feature of the surgical access system of FIG. 2;

FIG. 22 illustrates a generalized one-dimensional, two-electrode, direction-finding model;

FIG. 23 illustrates an electrode positioned along the x=y=0 z-axis, which is in a different x-y plane than a plurality of other electrodes;

FIG. 24 illustrates a first pair of electrodes in one plane, a second pair of electrodes in another plane and a nerve activation site;

FIG. 25 illustrates a device with four electrodes in a tetrahedron configuration;

FIG. 26 illustrates a device and a K-wire slidably received in the device with electrodes.

## DETAILED DESCRIPTION

Illustrative embodiments of the application are described below. In the interest of clarity, not all features of an actual implementation are described in this specification. It will of course be appreciated that in the development of any such actual embodiment, numerous implementation-specific decisions must be made to achieve the developers' specific goals, such as compliance with system-related and business-related constraints, which will vary from one implementation to another. Moreover, it will be appreciated that such a development effort might be complex and time-consuming, but would nevertheless be a routine undertaking for those of ordinary skill in the art having the benefit of this disclosure. The systems disclosed herein boast a variety of inventive features and components that warrant patent protection, both individually and in combination.

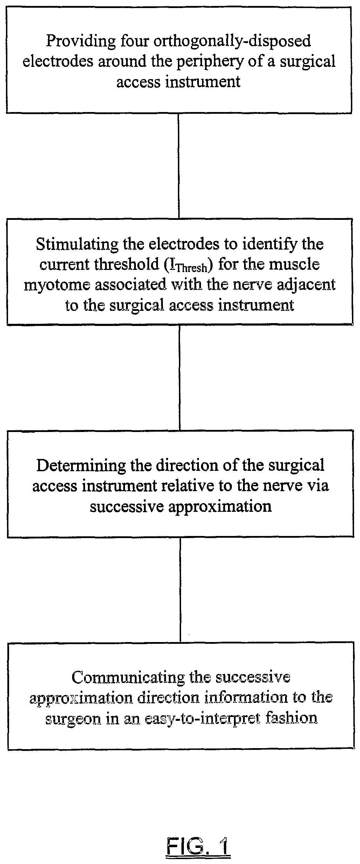

FIG. 1 illustrates general functions according to one embodiment of the present application, namely: (a) providing multiple (e.g., four orthogonally-disposed) electrodes around the periphery of the surgical access instrument; (b) stimulating the electrodes to identify the current threshold ($I_{Thresh}$) necessary to innervate the muscle myotome coupled to the nerve near the surgical access instrument; (c) determining the direction of the nerve relative to the surgical access instrument via successive approximation; and (d) communicating this successive approximation direction information to the surgeon in an easy-to-interpret fashion.

The act of providing multiple (e.g., four orthogonally-disposed) electrodes around the periphery of the surgical access instrument may be accomplished in any number of suitable fashions depending upon the surgical access instrument in question. For example, the electrodes may be disposed orthogonally on any or all components of a sequential dilation system (including an initial dilator, dilating cannulae, and working cannula), as well as speculum-type and/or retractor-based access systems, as disclosed in US2012/238893. The act of stimulating is accomplished by applying any of a variety of suitable stimulation signals to the electrode(s) on the surgical accessory, including current pulses of varying magnitude and/or frequency. The stimulating act may be performed during and/or after the process of creating an operative corridor to the surgical target site.

The act of determining the direction of the surgical access instrument relative to the nerve via successive approximation is performed by monitoring or measuring the EMG responses of muscle groups associated with a particular nerve and innervated by the nerve(s) stimulated during the process of gaining surgical access to a desired surgical target site.

The act of communicating this successive approximation information to the surgeon in an easy-to-interpret fashion may be accomplished in any number of suitable fashions, including but not limited to the use of visual indicia (such as alpha-numeric characters, light-emitting elements, and/or graphics) and audio communications (such as a speaker element). By way of example only, this may include providing an arc or other graphical representation that indicates the general direction to the nerve. The direction indicator may quickly start off relatively wide, become successively more narrow (based on improved accuracy over time), and may conclude with a single arrow designating the relative direction to the nerve.

The direction indicator may be an important feature. By providing such direction information, a user will be kept informed as to whether a nerve is too close to a given surgical accessory element during and/or after the operative corridor is established to the surgical target site. This is particularly advantageous during the process of accessing the surgical target site in that it allows the user to actively avoid nerves and redirect the surgical access components to successfully create the operative corridor without impinging or otherwise compromising the nerves.

Based on this nerve direction feature, then, an instrument is capable of passing through virtually any tissue with minimal (if any) risk of impinging or otherwise damaging associated neural structures within the tissue, thereby making the system suitable for a wide variety of surgical applications.

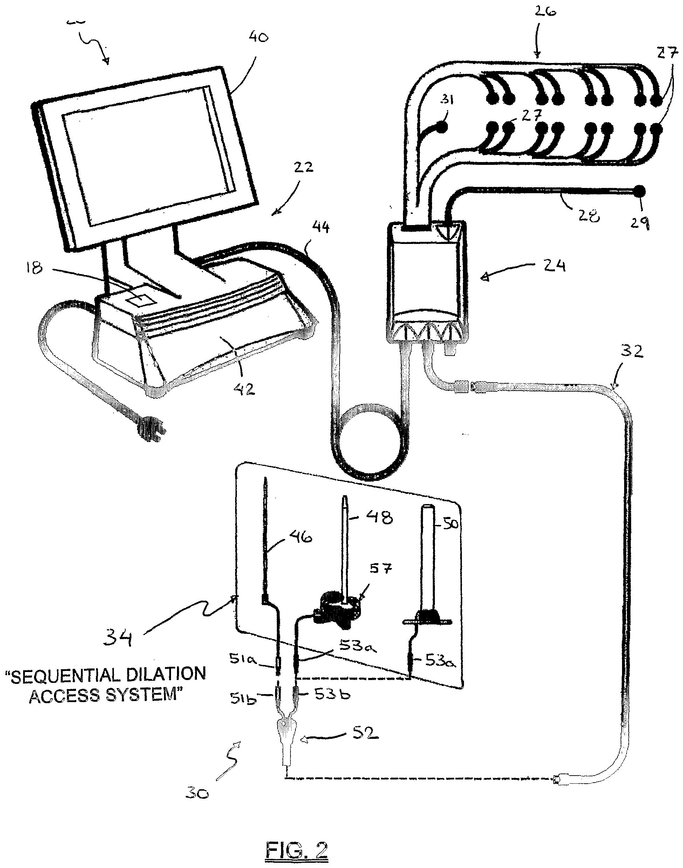

FIGS. 2-3 illustrate, by way of example only, a surgical system 20 provided in accordance with a broad aspect of the present application. The surgical system 20 includes a control unit 22, a patient module 24, an EMG harness 26 and return electrode 28 coupled to the patient module 24, and (by way of example only) a sequential dilation surgical access system 34 capable of being coupled to the patient module 24 via cable 32. The sequential dilation access system 34 comprises, by way of example only, a K-wire 46, one or more dilating cannula 48, and a working cannula 50.

The control unit 22 includes a touch screen display 40 and a base 42, which collectively contain the essential processing capabilities (software and/or hardware) for controlling the surgical system 20. The control unit 22 may include an audio unit 18 that emits sounds according to a location of a surgical element with respect to a nerve, as described herein.

The patient module 24 is connected to the control unit 22 via a data cable 44, which establishes the electrical connections and communications (digital and/or analog) between the control unit 22 and patient module 24. The main functions of the control unit 22 include receiving user commands via the touch screen display 40, activating stimulation electrodes on the surgical access instruments 30, processing signal data according to defined algorithms (described below), displaying received parameters and processed data, and monitoring system status and report fault conditions. The touch screen display 40 is preferably equipped with a graphical user interface (GUI) capable of communicating information to the user and receiving instructions from the user. The display 40 and/or base 42 may contain patient module interface circuitry (hardware and/or software) that commands the stimulation sources, receives digitized signals and other information from the patient module 24, processes the EMG responses to extract characteristic information for each muscle group, and displays the processed data to the operator via the display 40.

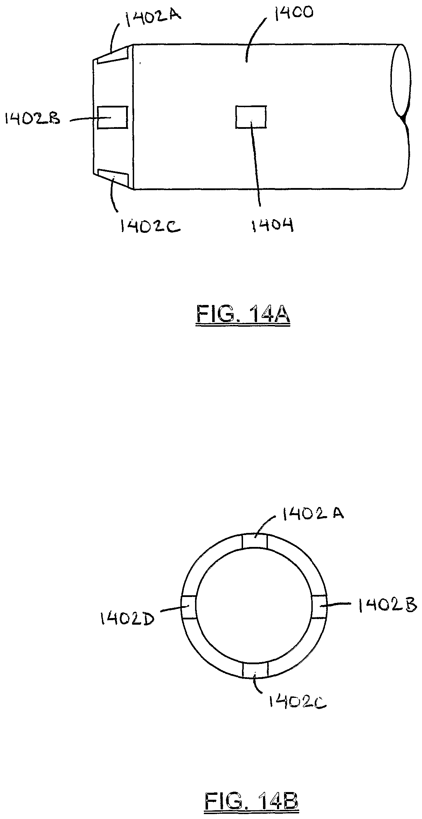

In one embodiment, the surgical system 20 is capable of determining nerve direction relative to each K-wire 46, dilation cannula 48 and/or the working cannula 50 during and/or following the creation of an operative corridor to a surgical target site. Surgical system 20 accomplishes this by having the control unit 22 and patient module 24 cooperate to send electrical stimulation signals to each of the orthogonally-disposed stimulation electrodes 1402A-1402D (FIGS. 14A-14B) on the various surgical access instruments 46-50 (e.g., electrodes on the distal ends of the instruments 46-50). Depending upon the location of the surgical access instruments 46-50 within a patient, the stimulation signals may cause nerves adjacent to or in the general proximity of the surgical instruments 46-50 to depolarize. This causes muscle groups to innervate and generate EMG responses, which can be sensed via the EMG harness 26. The nerve direction feature of the system 20 is based on assessing the evoked response of the various muscle myotomes monitored by the surgical system 20 via the EMG harness 26.

The sequential dilation surgical access system 34 is designed to bluntly dissect the tissue between the patient's skin and the surgical target site. Each K-wire 46, dilating cannula 48 and/or working cannula 50 may be equipped with multiple (e.g., four orthogonally-disposed) stimulation electrodes to detect the location of nerves in between the skin of the patient and the surgical target site. To facilitate this, a surgical hand-piece 52 is provided for electrically coupling the surgical accessories 46-50 to the patient module 24 (via cable 32). In a preferred embodiment, the surgical hand piece 52 includes one or more buttons for selectively initiating the stimulation signal (preferably, a current signal) from the control unit 22 to a particular surgical access instrument 46-50. Stimulating the electrode(s) on these surgical access instruments 46-50 during passage through tissue in forming the operative corridor will cause nerves that come into close or relative proximity to the surgical access instruments 46-50 to depolarize, producing a response in the innervated myotome.

By monitoring the myotomes associated with the nerves (via the EMG harness 26 and recording electrode 27) and assessing the resulting EMG responses (via the control unit 22), the sequential dilation access system 34 is capable of detecting the direction to such nerves. Direction determination provides the ability to actively negotiate around or past such nerves to safely and reproducibly form the operative corridor to a particular surgical target site. In one embodiment, by way of example only, the sequential dilation access system 34 is particularly suited for establishing an operative corridor to an intervertebral target site in a postero-lateral, trans-psoas fashion so as to avoid the bony posterior elements of the spinal column.

A discussion of the algorithms and principles behind the neurophysiology for accomplishing these functions will now be undertaken, followed by a detailed description of the various implementations of these principles.

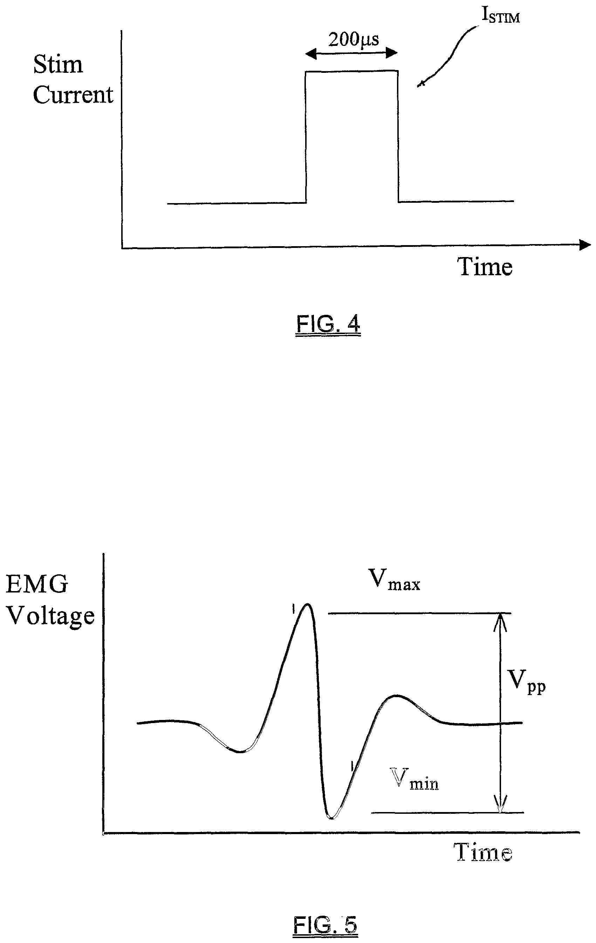

FIGS. 4 and 5 illustrate a fundamental aspect of the present application: a stimulation signal (FIG. 4) and a resulting evoked response (FIG. 5). By way of example only, the stimulation signal is preferably a stimulation current signal ($I_{Stim}$) having rectangular monophasic pulses with a frequency and amplitude adjustable by system software. In one embodiment, the stimulation current ($I_{Stim}$) may be coupled in any suitable fashion (i.e., AC or DC) and comprises rectangular monophasic pulses of 200 microsecond duration. The amplitude of the current pulses may be fixed, but may preferably sweep from current amplitudes of any suitable range, such as from 2 to 100 mA. For each nerve and myotome there is a characteristic delay from the stimulation current pulse to the EMG response (typically between 5 to 20 ms). To account for this, the frequency of the current pulses may be set at a suitable level, such as, in a preferred embodiment, 4 Hz to 10 Hz (and most preferably 4.5 Hz), so as to prevent stimulating the nerve before it has a chance to recover from depolarization. The EMG response shown in FIG. 5 can be characterized by a peak-to-peak voltage of $V_{pp} = V_{max} - V_{min}$.

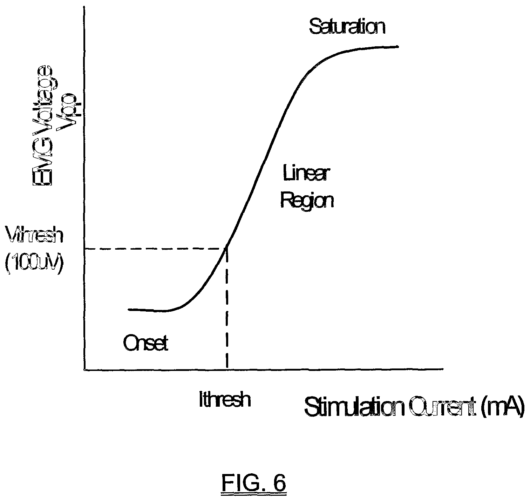

A basic premise behind the neurophysiology employed by the system 20 is that each nerve has a characteristic threshold current level ($I_{Thresh}$) at which it will depolarize. Below this threshold, current stimulation will not evoke a significant EMG response ($V_{pp}$). Once the stimulation threshold ($I_{Thresh}$) is reached, the evoked response is reproducible and increases with increasing stimulation until saturation is reached. This relationship between stimulation current and EMG response may be represented graphically via a so-called "recruitment curve," such as shown in FIG. 6, which includes an onset region, a linear region, and a saturation region. By way of example only, the system 20 may define a significant EMG response to have a Vpp of approximately 100 uV. In a preferred embodiment, the lowest stimulation current that evokes this threshold voltage ($V_{Thresh}$) is called a stimulation current threshold or "$I_{Thresh}$."

In order to obtain this useful information, the system 20 should first identify the peak-to-peak voltage (Vpp) of each EMG response that corresponds to a given stimulation current ($I_{Stim}$). The existence of stimulation and/or noise artifacts, however, can conspire to create an erroneous Vpp measurement of the electrically evoked EMG response. To overcome this challenge, the surgical system 20 may employ any number of suitable artifact rejection techniques. Having measured each Vpp EMG response (as facilitated by the stimulation and/or noise artifact rejection techniques), this Vpp information is then analyzed relative to the stimulation current in order to determine a relationship between the nerve and the given electrode on the surgical access instrument 46-50 transmitting the stimulation current. More specifically, the system 20 determines these relationships (between nerve and surgical accessory) by identifying the minimum stimulation current ($I_{Thresh}$) capable of producing a predetermined Vpp EMG response.

$I_{Thresh}$ may be determined for each of the four orthogonal electrodes 1402A-1402D (FIGS. 14A-14B) in an effort to determine the direction between the surgical access instrument 34, 36 and the nerve. This may be accomplished by employing a two-part threshold-hunting algorithm, including a bracketing process and a bi-section (or bisecting) process, which may proceed step-wise for each stimulation electrode to provide successive directional information to the user.

**Arc Method**

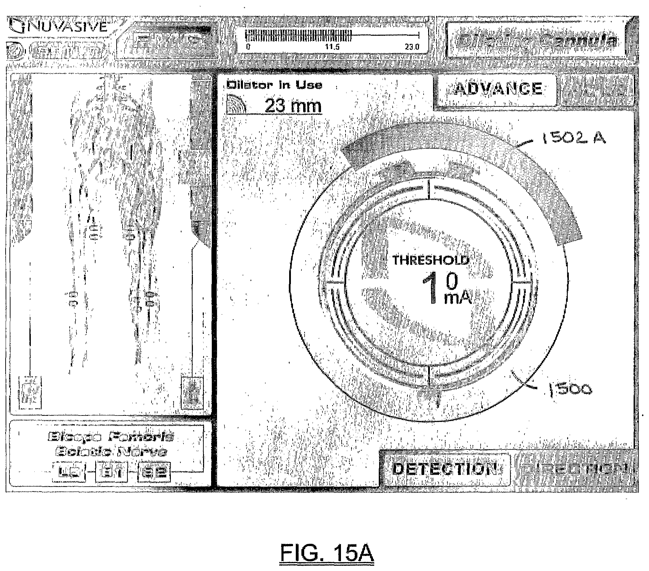

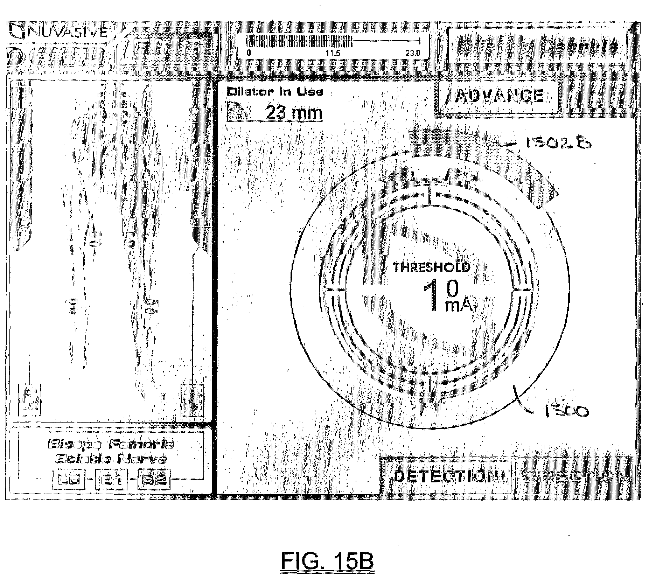

In one embodiment, successive directional information may take the form of an arc or wedge (or range) representing a zone that contains the nerve, according to an "arc" method described below. This successive directional information is based on stimulation current threshold "ranges," and may be displayed (FIGS. 15A-15C) or otherwise communicated to the surgeon any time the stimulation current thresholds are known to fall within a range of values.

In the bracketing process, an electrical stimulus is provided at each of the four orthogonal electrodes 1402A-1402D (FIGS. 14A-14B), beginning with a small current level (e.g. 0.2 mA) and ramping up. In the "arc" method, each of the four electrodes 1402A-1402D maybe stimulated at the same current level, in sequence, before proceeding to the next higher current level (as opposed to another method that completes the bracketing for one electrode 1402 before advancing to another electrode 1402). The goal is to identify a bracket around the stimulation current for each of the four stimulation electrodes 1402A-1402D. If a stimulation current threshold has been bracketed for an electrode 1402, the bracketing act is complete for that electrode 1402, and stimulation proceeds for the remaining electrodes until the stimulation current threshold has been bracketed for each electrode. As the bracketing process proceeds, each new stimulation provides information about the "range" of the current threshold for that electrode 1402. This information may bracket the stimulation current threshold (e.g., between 1.6 and 3.2 mA), or it may only provide a lower bound for the current threshold (e.g., threshold is greater than 5.0 mA). In either event, the "arc" bracketing process proceeds for each of the stimulation electrodes 1402A-1402D to provide, in succession, more accurate information regarding the direction of the nerve relative to the surgical access instrument 46-50.

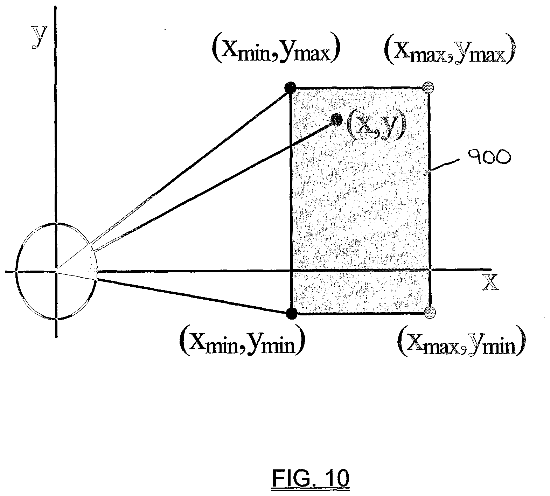

As shown in FIG. 10, an arc (wedge) containing the final direction vector is computed from the range information for the stimulation current thresholds corresponding to the four stimulation electrodes 1402A-1402D. This can be done as often as desired as the bracketing method proceeds. The arc (wedge) may then be used to display directional information to the operator, as in FIGS. 15A-15C.

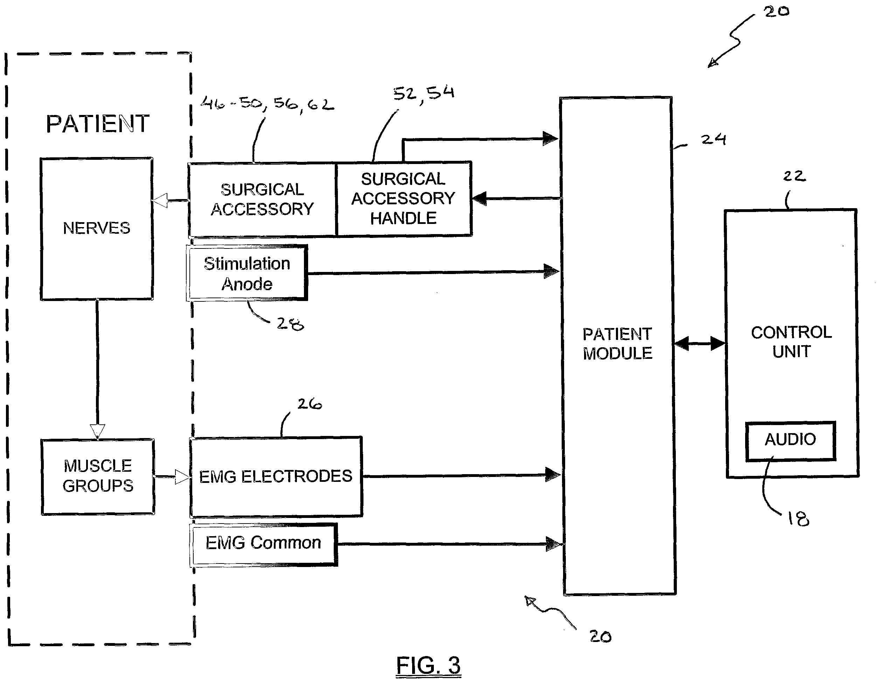

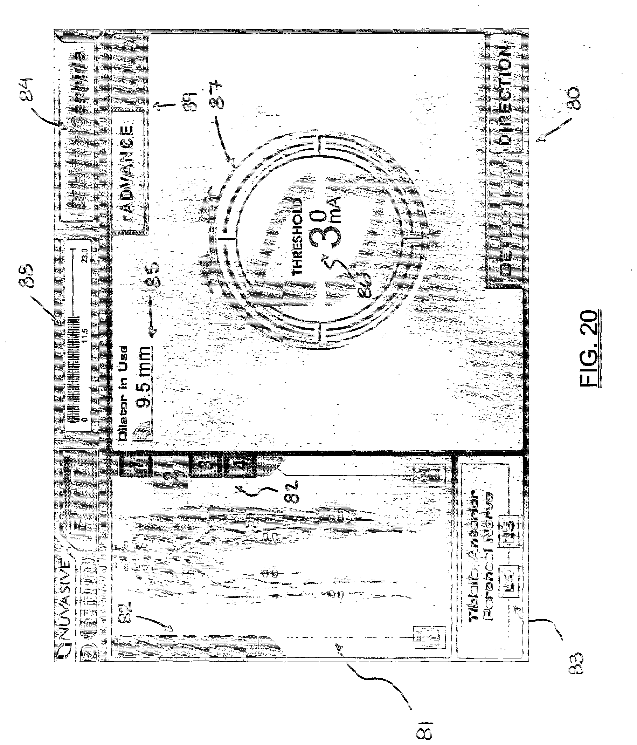

This successive approximation information may be communicated to the surgeon in a number of easy-to-interpret fashions, including but not limited to the use of visual indicia (such as alpha-numeric characters, light-emitting elements, and/or graphics, as in FIGS. 15A-15C and 20-21) and audio communications (such as a speaker element 18 in FIG. 3). By way of example only, this successive directional information may include providing an "arc" 1502 (hence the name "arc" method) or other graphical representation that indicates the general direction to the nerve, which may start off relatively wide, become successively more narrow (based on improved accuracy over time), and may conclude with a single arrow designating the relative direction to the nerve. FIGS. 15A-15C illustrate screenshots of a cross-section of an instrument 1500 and a wide direction arc 1502A, a narrower direction arc 1502B and an arrow 1502C as more stimulation current pulses are generated and EMG responses are analyzed during the bracketing and bisecting processes.

There are a number of possibilities for displaying the arc information. An arc or wedge might be displayed. Alternatively, an arrow might point to the midpoint of the arc. Another indicator might be used to illustrate the width of the arc (i.e. the uncertainty remaining in the result).

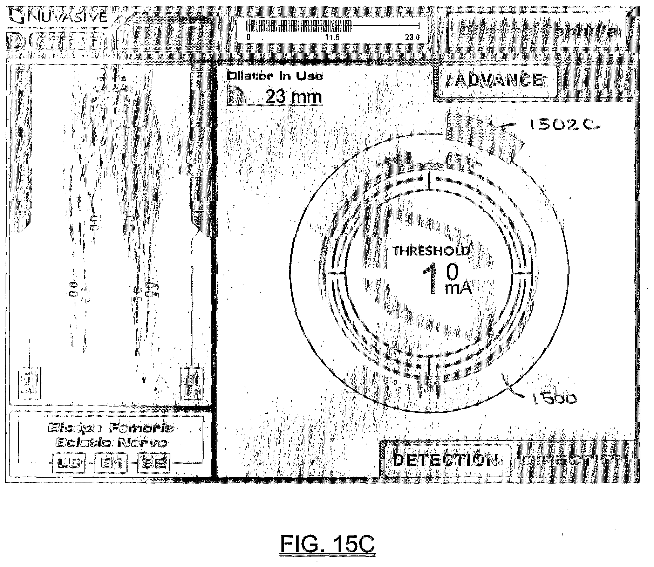

Upon completion of the bracketing process, a bisection process may determine more precisely the stimulation current thresholds. As with the bracketing process, current stimulations may be "rotated" among the stimulation electrodes so that the thresholds are refined substantially in parallel, according to the "arc" method. As with the bracketing method, the arc (wedge) 1502 containing the final direction vector may be computed and displayed (FIGS. 15A-15C) frequently during the process. Upon completion of the bisection method for all electrodes 1402A-1402D, the stimulation current thresholds are identified precisely. At that time, the final direction vector 1502C (FIG. 15C and FIGS. 20-21) maybe displayed.

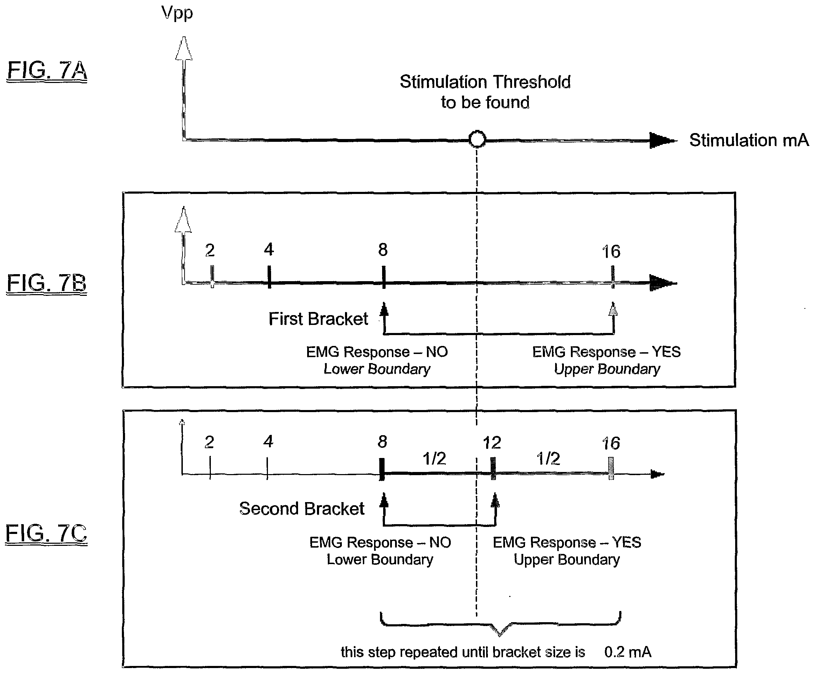

The above-identified two-part hunting-algorithm may be further explained with reference to FIGS. 7A-7E. According to the arc method, each electrode 1402 is stimulated at the same stimulation current level before passing to the next stimulation current level. In this fashion, successive directional information can be obtained as described above. Threshold current ($I_{Thresh}$) is the minimum stimulation current ($I_{Stim}$) (FIG. 6) that produces a Vpp (FIG. 5) greater than a known threshold voltage ($V_{Thresh}$). The value of $I_{Stim}$ may be adjusted by a bracketing method as follows. The first bracket may be 0.2 mA and 0.3 mA. If the Vpp corresponding to both of these stimulation currents is lower than Vthresh, then the bracket size may be doubled to 0.2 mA and 0.4 mA. This doubling of the bracket size continues until the upper end of the bracket results in a Vpp that is above $V_{Thresh}$.

The size of the brackets may then be reduced by a bisection method. A current stimulation value at the midpoint of the bracket is used, and if this results in a Vpp that is above Vthresh, then the lower half becomes the new bracket. Likewise, if the midpoint Vpp is below Vthresh, then the upper half becomes the new bracket. This bisection method is used until the bracket size has been reduced to $I_{Thresh}$ mA. $I_{Thresh}$ may be selected as a value falling within the bracket, but is preferably defined as the midpoint of the bracket.

The threshold-hunting algorithm of this embodiment may support three states: bracketing, bisection, and monitoring. A "stimulation current bracket" is a range of stimulation currents that bracket the stimulation current threshold $I_{Thresh}$. The width of a bracket is the upper boundary value minus the lower boundary value. If the stimulation current threshold $I_{Thresh}$ of a channel exceeds the maximum stimulation current, that threshold is considered out-of-range. During the bracketing state, threshold hunting will employ the method described herein to select stimulation currents and identify stimulation current brackets for each EMG channel in range.

The initial bracketing range may be provided in any number of suitable ranges. In one embodiment, the initial bracketing range is 0.2 to 0.3 mA. If the upper stimulation current does not evoke a response, the upper end of the range should be increased. For example, the range scale factor may be 2. The stimulation current should preferably not be increased by more than 10 mA in one iteration. The stimulation current should preferably never exceed a programmed maximum stimulation current (to prevent nerve damage, injury or other undesirable effects). For each stimulation, the algorithm will examine the response of each active channel to determine whether the stimulation current falls within that bracket. Once the stimulation current threshold of each channel has been bracketed, the algorithm transitions to the bisection state.

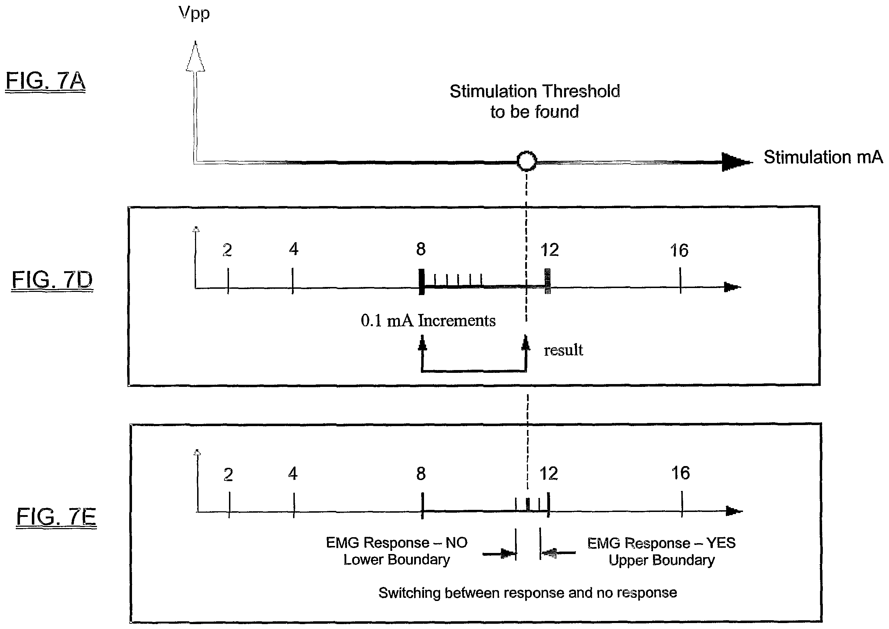

During the bisection state (FIGS. 7C and 7D), threshold hunting may select stimulation currents and narrow the bracket to a selected width (for example, 0.1 mA) for each EMG channel with an in-range threshold. After the minimum stimulation current has been bracketed (FIG. 7B), the range containing the root is refined until the root is known with a specified accuracy. The bisection method is used to refine the range containing the root. In one embodiment, the root should be found to a precision of 0.1 mA. During the bisection method, the stimulation current at the midpoint of the bracket is used. If the stimulation evokes a response, the bracket shrinks to the lower half of the previous range. If the stimulation fails to evoke a response, the bracket shrinks to the upper half of the previous range. The nerve proximity/direction detection algorithm is locked on the electrode position when the response threshold is bracketed by stimulation currents separated by the selected width (i.e., 0.1 mA). The process is repeated for each of the active channels until all thresholds are precisely known. At that time, the algorithm may enter the monitoring state.

During the monitoring state (FIG. 7E), threshold hunting may employ the method described below to select stimulation currents and identify whether stimulation current thresholds are changing. In the monitoring state, the stimulation current level may be decremented or incremented by 0.1 mA, depending on the response of a specific channel. If the threshold has not changed, then the lower end of the bracket should not evoke a response, while the upper end of the bracket should. If either of these conditions fail, the bracket is adjusted accordingly. The process is repeated for each of the active channels to continue to assure that each threshold is bracketed. If stimulations fail to evoke the expected response three times in a row, then the algorithm may transition back to the bracketing state in order to reestablish the bracket.

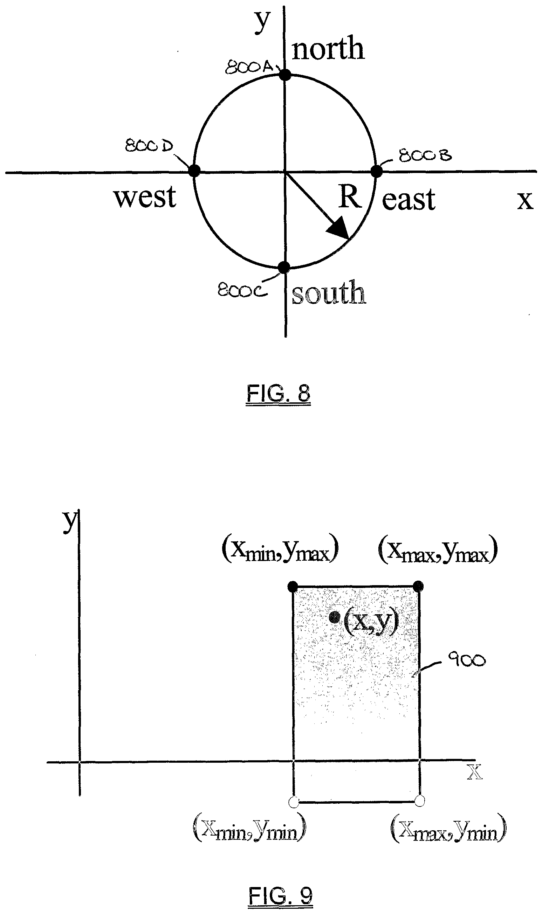

A method for computing the successive arc/wedge directional information from stimulation current threshold range information is described. The stimulation current threshold is presumed to be proportional to a distance to the nerve. The nerve may be modeled as a single point. Since stimulation current electrodes are in an orthogonal array, calculation of the X- and Y-dimension components of the direction vector may proceed independently. With reference to FIG. 8, the North and South electrodes 800A, 800C contribute to the Y-dimension component, while the East and West electrodes 800B-800D contribute to the X-dimension component. The direction vector <x, y> to a nerve may be defined as:

$$x = i_w^2 - i_e^2 \qquad (1)$$

$$y = i_s^2 - i_n^2$$

where $i_e$, $i_w$, $i_n$, and $i_s$ represent the stimulation current thresholds for the east, west, north, and south electrodes 802B, 802D, 802A, 802C, respectively. (The equations may be normalized to an arbitrary scale for convenience.)

As the threshold hunting method begins, the stimulation current thresholds are known only within a range of values. Therefore, the X- and Y-dimension components are known only within a range. This method provides an extension to the previous definitions, as follows:

$$x_{\min} = i_{w,\min}^2 - i_{e,\max}^2$$

$$x_{\max} = i_{w,\max}^2 - i_{e,\min}^2 \qquad (2)$$

$$y_{\min} = i_{s,\min}^2 - i_{n,\max}^2$$

$$y_{\max} = i_{s,\max}^2 - i_{n,\min}^2$$

Just as $i_{e,\min}$ and $i_{e,\max}$ bracket $i_e$, x and y are bracketed by $[x_{\min}, x_{\max}]$ and $[y_{\min}, y_{\max}]$. Stated another way, the point (x,y) lies within a rectangle 900 described by these boundaries, as shown in FIG. 9. As shown in FIG. 10, just as the point (x,y) represents a vector from the origin to a nerve modeled as a point, the bounding rectangle 900 represents an arc (wedge) containing that vector.

The arc method may have several advantages. First, the arc is capable of narrowing in a relatively quick fashion as more stimulations and responses are analyzed. This method provides general directional information much faster than if the current threshold for each electrode 1402 was determined before moving on the next electrode 1402. With general directional information, it may be possible to terminate the stimulation before having the ultimate precision of the stimulation current vectors. This will result in a faster response, in many instances. The arc method may provide a real-time view of the data-analysis. This helps illustrate the value of additional stimulations to a user. This educates and empowers the user. The user can observe the progress of this method, which aids in the understanding of the time the system 20 takes to converge on the final direction vector. More frequent display updates help time "go faster" for the user. This method avoids a long pause that might seem even longer. Disclosure of the intermediate acts (narrowing arcs) in the process of finding the direction vector invites mutual trust between the user and the access system 30. An arc may provide a more intuitive visualization for neural tissue than a direction vector.

The arc method may also be useful in tracking direction as the instrument and stimulation electrodes move relative to the nerve. For example, if the uncertainty in the stimulation current threshold increases, this can be reflected in an increasing arc size.

The sequential dilation access system 34 (FIG. 2) of the system 20 is capable of accomplishing safe and reproducible access to a surgical target site. It does so by detecting the existence of and direction to neural structures before, during, and after the establishment of an operative corridor through (or near) any of a variety of tissues having such neural structures. If neural structures are contacted or impinged, this may result in neural impairment for the patient.

In one embodiment, the surgical system 20 accomplishes this through the use of the surgical hand-piece 52, which may be electrically coupled to the K-wire 46 via a first cable connector 51a, 51b and to either the dilating cannula 48 or the working cannula 50 via a second cable connector 53a, 53b. For the K-wire 46 and working cannula 50, cables are directly connected between these accessories and the respective cable connectors 51a, 53a for establishing electrical connection to the stimulation electrode(s). In one embodiment, a pincher or clamp-type device 57 is provided to selectively establish electrical communication between the surgical hand-piece 52 and the stimulation electrode(s) on the distal end of the cannula 48. This is accomplished by providing electrical contacts on the inner surface of the opposing arms forming the clamp-type device 57, wherein the contacts are dimensioned to be engaged with electrical contacts (preferably in a male-female engagement scenario) provided on the dilating cannula 48 and working cannula 50. The surgical hand-piece 52 includes one or more buttons such that a user may selectively direct a stimulation current signal from the control unit 22 to the electrode(s) on the distal ends of the surgical access components 46-50. In an important aspect, each surgical access component 46-50 is insulated along its entire length, with the exception of the electrode(s) at their distal end. In the case of the dilating cannula 48 and working cannula 50, the electrical contacts at their proximal ends for engagement with the clamp 57 are not insulated. The EMG responses corresponding to such stimulation may be monitored and assessed in order to provide nerve proximity and/or nerve direction information to the user.

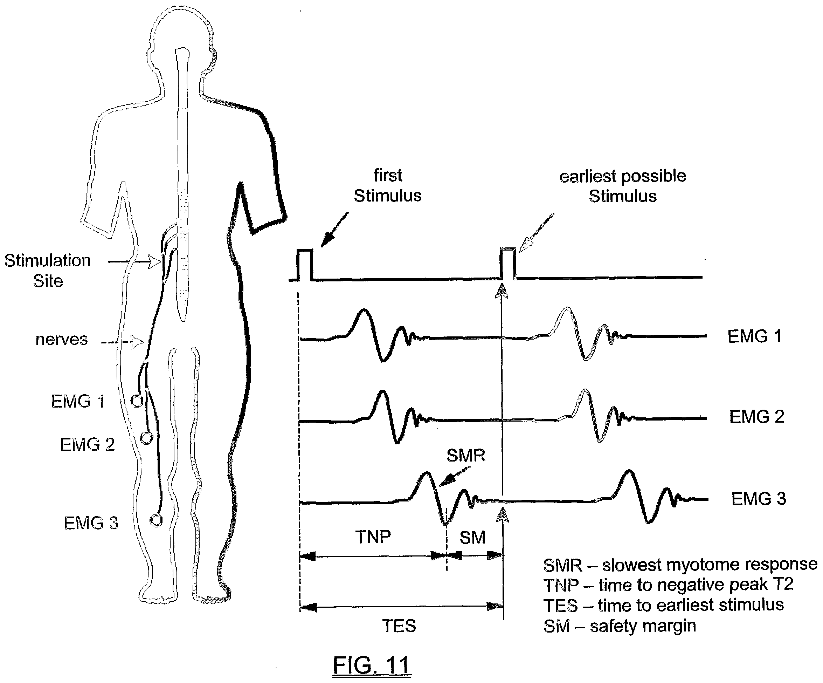

When employed in spinal procedures, for example, such EMG monitoring would preferably be accomplished by connecting the EMG harness 26 to the myotomes in the patient's legs corresponding to the exiting nerve roots associated with the particular spinal operation level (see FIGS. 11 and 20-21). In a preferred embodiment, this is accomplished via 8 pairs of EMG electrodes 27 (FIG. 2) placed on the skin over the major muscle groups on the legs (four per side), an anode electrode 29 providing a return path for the stimulation current, and a common electrode 31 providing a ground reference to pre-amplifiers in the patient module 24. Although not shown, it will be appreciated that any of a variety of electrodes can be employed, including but not limited to needle electrodes. The EMG responses measured via the EMG harness 26 provide a quantitative measure of the nerve depolarization caused by the electrical stimulus. By way of example, the placement of EMG electrodes 27 may be undertaken according to the manner shown in Table 1 below for spinal surgery:

Table 1

| Color | Channel ID | Myotome | Spinal Level |
|-------|-----------|---------|-------------|
| Blue | Right 1 | Right Vastus Medialis | L2, L3, L4 |
| Violet | Right 2 | Right TibialisAnterior | L4, L5 |
| Grey | Right 3 | Right Biceps Femoris | L5, S1, S2 |
| White | Right 4 | Right Gastroc. Medial | S1, S2 |
| Red | Left 1 | Left Vastus Medialis | L2, L3, L4 |
| Orange | Left 2 | Left Tibialis Anterior | L4, L5 |
| Yellow | Left 3 | Left Biceps Femoris | L5, S1, S2 |
| Green | Left 4 | Left Gastroc. Medial | S1, S2 |

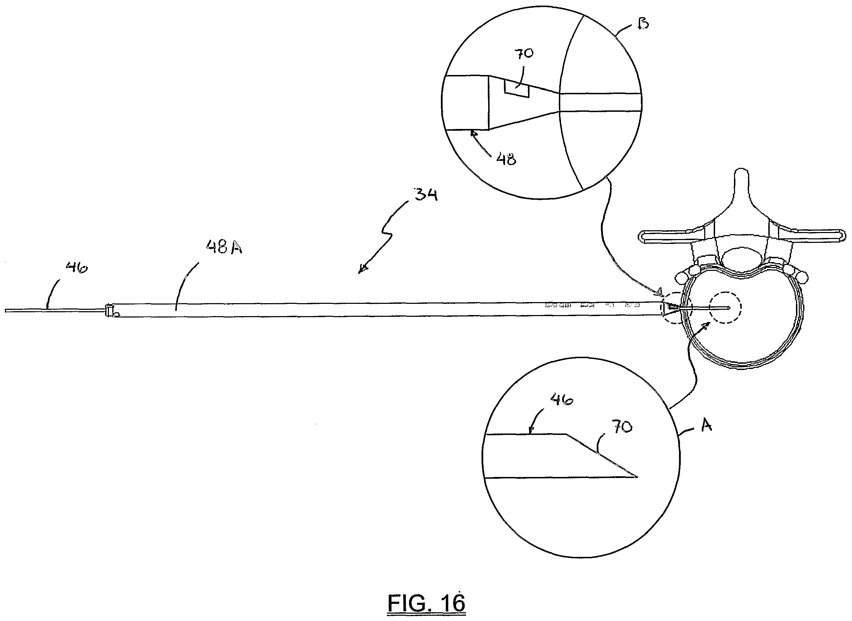

FIGS. 16-19 illustrate the sequential dilation access system 34 in FIG. 2 in use creating an operative corridor to an intervertebral disk. As shown in FIG. 16, an initial dilating cannula 48A is advanced towards the target site with the K-wire 46 disposed within an inner lumen within the dilating cannula 48. This may be facilitated by first aligning the K-wire 46 and initial dilating cannula 48A using any number of commercially available surgical guide frames. In one embodiment, as best shown in the expanded insets A and B, the K-wire 46 and initial dilating cannula 48A are each equipped with a single stimulation electrode 70 to detect the presence and/or location of nerves in between the skin of the patient and the surgical target site. More specifically, each electrode 70 may be positioned at an angle relative to the longitudinal axis of the K-wire 46 and dilator 48 (and working cannula 50). In one embodiment, this angle may range from 5 to 85 degrees from the longitudinal axis of these surgical access components 46-50. By providing each stimulation electrode 70 in this fashion, the stimulation current will be directed angularly from the distal tip of the respective accessory 46, 48. This electrode configuration is advantageous in determining proximity, as well as direction, according to the present application in that a user may simply rotate the K-wire 46 and/or dilating cannula 48 while stimulating the electrode 70. This may be done continuously or step-wise, and preferably while in a fixed axial position. In either case, the user will be able to determine the location of nerves by viewing the proximity information on the display screen 40 and observing changes as the electrode 70 is rotated. This may be facilitated by placing a reference mark 72 on the K-wire 46 and/or dilator 48 (or a control element coupled thereto), indicating the orientation of the electrode 70 to the user.

In another embodiment, the K-wire 46 and dilating cannula 48 in FIG. 2 may each have multiple electrodes, as described above and shown in FIGS. 14A-14B.

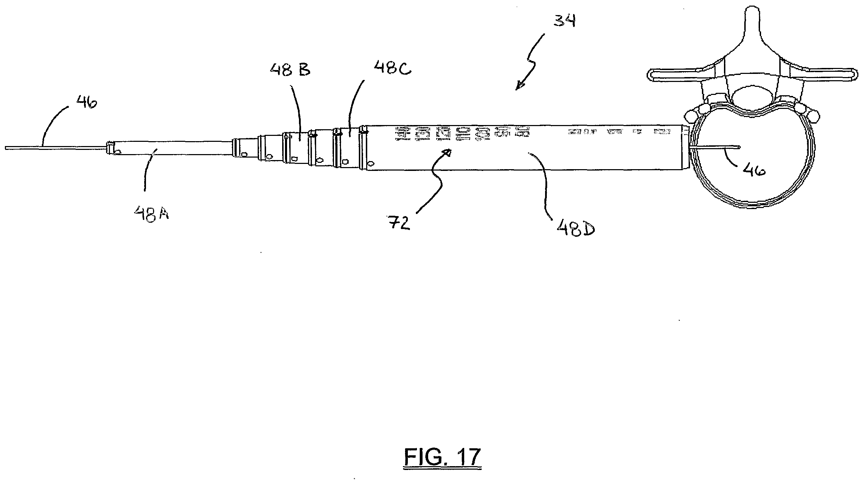

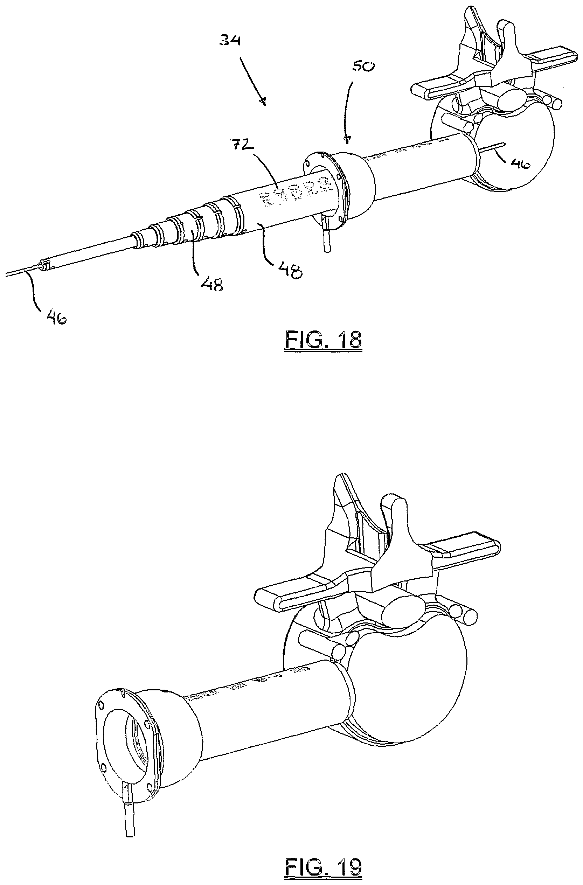

In the embodiment shown, the trajectory of the K-wire 46 and initial dilator 48A is such that they progress towards an intervertebral target site in a postero-lateral, trans-psoas fashion so as to avoid the bony posterior elements of the spinal column. Once the K-wire 46 is docked against the annulus of the particular intervertebral disk, cannulae of increasing diameter 48B-48D may then be guided over the previously installed cannula 48A (sequential dilation) until a desired lumen diameter is installed, as shown in FIG. 17. By way of example only, the dilating cannulae 48A-48D may range in diameter from 6 mm to 30 mm, with length generally decreasing with increasing diameter size. Depth indicia 72 may be optionally provided along the length of each dilating cannula 48 to aid the user in gauging the depth between the skin of the patient and the surgical target site. As shown in FIG. 18, the working cannula 50 may be slideably advanced over the last dilating cannula 48D after a desired level of tissue dilation has been achieved. As shown in FIG. 19, the last dilating cannula 48D and then all the dilating cannulae 48 may then be removed from inside the inner lumen of the working cannula 50 to establish the operative corridor therethrough.

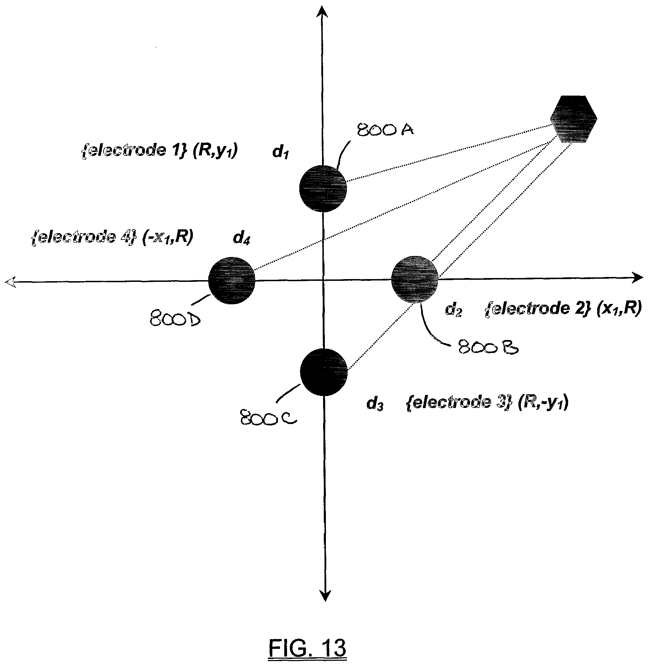

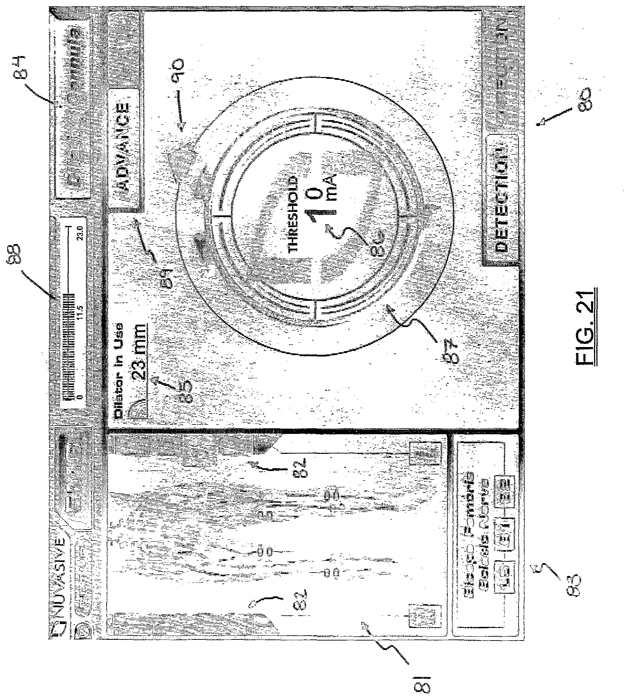

Once a nerve is detected using the K-wire 46, dilating cannula 48, or the working cannula 50, the surgeon may select the DIRECTION function to determine the angular direction to the nerve relative to a reference mark on the access components 46-50, as shown in FIG. 21. In one embodiment, a directional arrow 90 is provided, by way of example only, disposed around the cannula graphic 87 for the purpose of graphically indicating to the user which direction the nerve is relative to the access components 46-50. This information helps the surgeon avoid the nerve as he or she advances the K-wire 46 and cannulae 48, 50. In one embodiment, this directional capability is accomplished by equipping the K-wire 46, dilators 48 and working cannula 50 with four (4) stimulation electrodes disposed orthogonally on their distal tip (FIGS. 14A-14B). These electrodes are preferably scanned in a monopolar configuration (that is, using each of the 4 electrodes as the stimulation source). The threshold current ($I_{thresh}$) is found for each of the electrodes by measuring the muscle evoked potential response Vpp and comparing it to a known threshold Vthresh. From this information, the direction from a stimulation electrode (or device 46-50) to a nerve may be determined according to the algorithm and technique described herein and with reference to FIGS. 8-10, 13, 14A-14B and 15A-15C. In FIGS. 8 and 13, the four (4) electrodes 800A-800D are placed on the x and y axes of a two dimensional coordinate system at a radius R from the origin. A vector is drawn from the origin along the axis corresponding to each electrode. Each vector has a length equal to $I_{Thresh}$ for that electrode. Thus, with four electrodes 800A-800D, four vectors are drawn from the origin along the four axi corresponding to the four electrodes. The vector from the origin to a direction pointing toward the nerve is then computed. Using the geometry shown, the (x,y) coordinates of the nerve, taken as a single point, can be determined as a function of the distance from the nerve to each of four electrodes 800A-800D. This can be expressly mathematically as follows:

Where the "circles" in FIG. 13 denote the position of the electrode respective to the origin or center of the cannula, the "hexagon" denotes the position of a nerve, and $d_1$, $d_2$, $d_3$, and $d_4$ denote the distance between the nerve point and stimulation electrodes 1-4 (north, east, south and west) respectively, it can be shown that:

$$y = \frac{d_1^2 - d_3^2}{-4R} \quad \text{and} \quad x = \frac{d_2^2 - d_4^2}{-4R}$$

Where R is the cannula radius, standardized to 1, since angles and not absolute values are measured.

After conversion from Cartesian coordinates (x,y) to polar coordinates (r,$\theta$), then $\theta$ is the angular direction to the nerve. This angular direction may then be displayed to the user, by way of example only, as the arrow 90 shown in FIG. 21 pointing towards the nerve. In this fashion, the surgeon can actively avoid the nerve, thereby increasing patient safety while accessing the surgical target site. The surgeon may select any one of the 4 channels available to perform the Direction Function, although the channel with the lowest stimulation current threshold (that indicates a nerve closest to the instrument) should probably be used. The surgeon should preferably not move or rotate the instrument while using the Direction Function, but rather should return to the Detection Function to continue advancing the instrument.

After establishing an operative corridor to a surgical target site via the surgical access system 34, any number of suitable instruments and/or implants may be introduced into the surgical target site depending upon the particular type of surgery and surgical need. By way of example only, in spinal applications, any number of implants and/or instruments may be introduced through the working cannula 50, including but not limited to spinal fusion constructs (such as allograft implants, ceramic implants, cages, mesh, etc.), fixation devices (such as pedicle and/or facet screws and related tension bands or rod systems), and any number of motion-preserving devices (including but not limited to total disc replacement systems).

**Segregating the Geometric and Electrical Direction Algorithm Models**

There are other relationships resulting from the symmetry of the four electrodes described above:

$$(1) \qquad d_w^2 + d_e^2 = d_s^2 + d_n^2$$

and

$$(2) \qquad d_0^2 + R^2 = \frac{1}{4}\left(d_w^2 + d_e^2 + d_s^2 + d_n^2\right)$$

where $d_0$ is the distance between the nerve activation site and the midpoint between the electrodes (i.e., the origin (0, 0) or "virtual center"). These results are based purely on geometry and apply independent of an electrical model.

As described above under the "arc" method, the geometric model can be extended to define a region of uncertainty based on the uncertainty in the distance to the nerve:

$$(3) \qquad x_{\min} = \frac{1}{4R}\left(d_{w,\min}^2 - d_{e,\max}^2\right)$$

$$x_{\max} = \frac{1}{4R}\left(d_{w,\max}^2 - d_{e,\min}^2\right)$$

$$y_{\min} = \frac{1}{4R}\left(d_{s,\min}^2 - d_{n,\max}^2\right)$$

$$y_{\max} = \frac{1}{4R}\left(d_{s,\max}^2 - d_{n,\min}^2\right)$$

In FIGS. 8 and 13, the two-dimensional x-y model assumes that the nerve activation site lies in the same plane as the stimulation electrodes or the entire z-axis space may be considered to be projected onto the x-y plane. It has been found that the z-dimension has no effect on direction "in the plane." The distance equations presented in the previous sections also apply when the nerve activation site is out of the plane of the stimulation electrodes.

**Generalized 1-D Model**

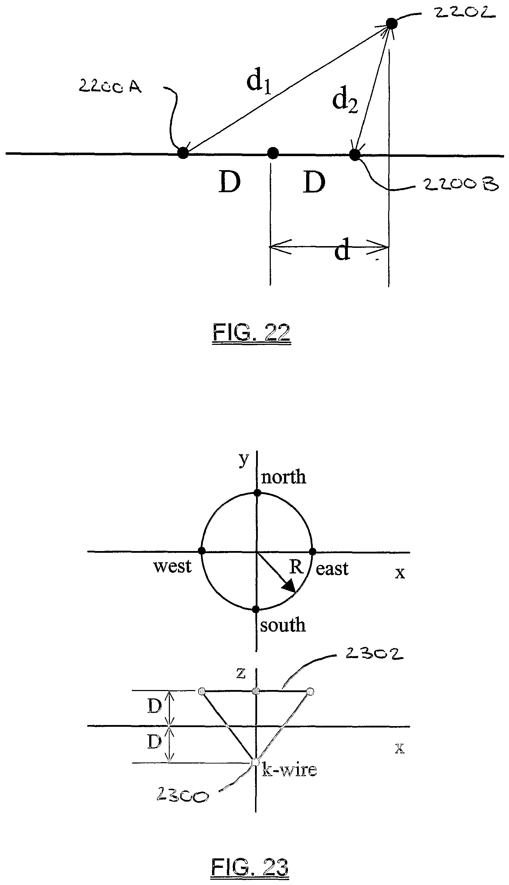

FIG. 22 illustrates two electrodes 2200A, 2200B. Given any two electrodes 2200A, 2200B, the absolute position of the nerve activation site 2202 in one dimension can be computed from the distances from those two electrodes:

$$(4) \qquad d = \frac{1}{4D}\left(d_1^2 - d_2^2\right)$$

The 1-D model can be extended to two or three dimensions by the addition of electrodes.

**3-D Geometric Model**

Using the four co-planar electrodes (FIGS. 8 and 13), it is not particularly easy to identify direction to the nerve along the z-axis. This may be easily rectified by the addition of one or more stimulation electrodes 2300 (FIG. 23) out of the original x-y electrode plane. For example, FIG. 23 shows a k-wire electrode 2300, positioned along the x=y=0 z-axis. D is half the distance between the K-wire electrode 2300 and the plane 2302 of the other four electrodes.

3-D direction to the nerve is possible by comparing the distance to the nerve activation site from the k-wire electrode 2300 to that of the other electrodes.

$$(5) \qquad z = \frac{1}{4D}\left(d_o^2 - d_k^2\right)$$

where $d_o$ (as noted above) is the distance between the nerve activation site and the midpoint between the electrodes (i.e., the origin (0, 0) or "virtual center"), and $d_k$ is the distance between the nerve activation site and the k-wire electrode 2300. 3-D direction is possible by converting from Cartesian (x, y, z) to spherical ($\rho$, $\theta$, $\varphi$) coordinates. The arc method described above may also be extended to three dimensions. Other 3-D geometric models may be constructed. One possibility is to retain the four planar electrodes 1402A-1402D and add a fifth electrode 1404 along the side of the cannula 1400, as shown in FIG. 14A.

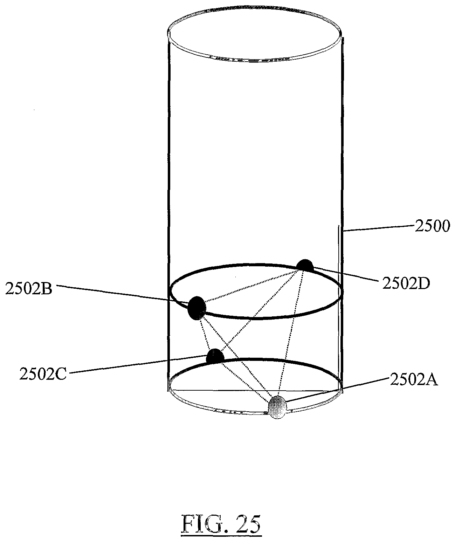

Another possibility is to replace the four planar electrodes 2502A-2502D with two pairs (e.g., vertices of a tetrahedron), as shown in FIG. 25. FIG. 25 illustrates a device 2500 with four electrodes 2502A-2502D in a tetrahedron configuration, which may be used with the system 20. Four electrodes may be a minimum for spanning a 3-D space, and may be the most efficient in terms of number of stimulations required to find the stimulation current thresholds.

**Electric Model**

The direction algorithm described above assumes direct proportionality between distance and the stimulation current threshold, as in equation (2).

$$(6) \qquad i_{th} = Kd$$

where $i_{th}$ is the threshold current, K is a proportionality constant denoting a relationship between current and distance, and d is the distance between an electrode and a nerve.

An alternative model expects the stimulation current threshold to increase with the square of distance:

$$(7) \qquad i_{th} = i_o + Kd^2$$

Using the distance-squared model, the Cartesian coordinates for the nerve activation site can be derived from equations (5), (7) and the following:

$$x = \frac{1}{4R}\left(d_w^2 - d_e^2\right)$$

$$y = \frac{1}{4R}\left(d_s^2 - d_n^2\right)$$

$$(8) \qquad x = \frac{1}{4RK}\left(i_w - i_e\right)$$

$$y = \frac{1}{4RK}\left(i_s - i_n\right)$$

$$z = \frac{1}{4DK}\left(i_c - i_k\right)$$

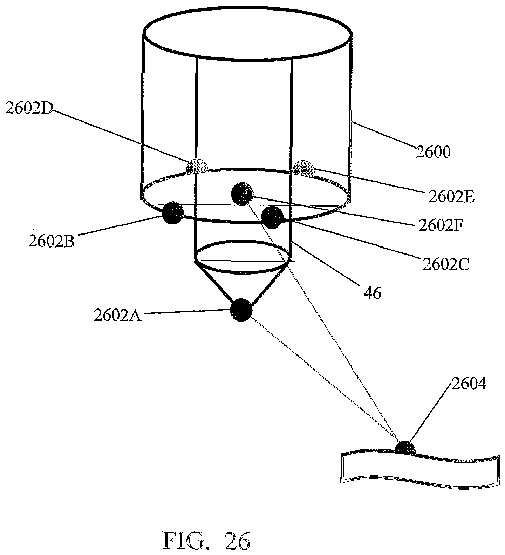

where $i_x$ is the stimulation current threshold of the corresponding stimulation electrode (west, east, south or north), $i_k$ is the stimulation current threshold of the k-wire electrode 2602A (FIG. 26), and $i_c$ is calculated from:

$$(9) \qquad i_c + KR^2 = \frac{1}{4}\left( i_w + i_e + i_s + i_n \right)$$

FIG. 26 illustrates a device 2600, such as a cannula 48A in FIG. 16, and a K-wire 46 slidably received in the device 2600. Both the K-wire 46 and the device 2600 have electrodes 2602A-2602F.

Other sets of equations may be similarly derived for alternative electrode geometries. Note that in each case, $i_0$ is eliminated from the calculations. This suggests that the absolute position of the nerve activation site relative to the stimulation electrodes may be calculated knowing only K. As noted above, K is a proportionality constant denoting the relationship between current and distance.

Distance or position of the neural tissue may be determined <u>independent</u> of nerve status or pathology (i.e., elevated $i_0$), so long as stimulation current thresholds can be found for each electrode.

### Measuring Nerve Pathology

If the distance to the nerve is known (perhaps through the methods described above), then it is possible to solve equation (8) for $i_0$. This would permit detection of nerves with elevated stimulation thresholds, which may provide useful clinical (nerve pathology) information.

$$(10) \qquad i_o = i_{th} - Kd^2$$

### Removing Dependence on K

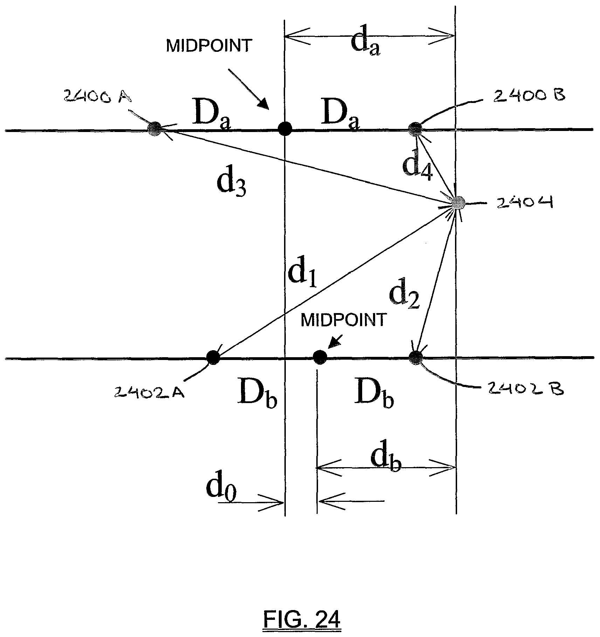

The preceding descriptions assume that the value for K is known. It is also possible to measure distance to a nerve activation site without knowing K, by performing the same measurement from two different electrode sets. FIG. 24 illustrates two pairs of electrodes 2400A, 2400B, 2402A, 2402B and a nerve activation site 2404. The top two electrodes 2400A, 2400B form one pair, and the bottom two electrodes 2402A, 2402B form a second pair. Using the electrodes 2400A, 2400B, 2402A, 2402B and distances defined in FIG. 24, the geometric model from equation (4) becomes:

$$(11) \qquad \frac{d_a}{d_b} = \frac{D_b}{D_a}\left( \frac{d_3^2 - d_4^2}{d_1^2 - d_2^2} \right) = \frac{d_0 + d_b}{d_b} = \frac{d_0}{d_b} + 1$$

Adding the electrical model from equation (7), the dependence on K is removed. Solve equation (11) for $d_b$ to get the distance in one dimension:

$$(12) \qquad \frac{D_b}{D_a}\left( \frac{i_3 - i_4}{i_1 - i_2} \right) = \frac{d_0}{d_b} + 1$$

Finally, it is possible to solve for the value of K itself:

$$(13) \qquad K = \frac{i_1 - i_2}{4D_b d_b}$$

Although the configuration in FIG. 14 shows four electrodes, the technique may also work with three collinear electrodes.

### Electrode Redundancy

Whichever electrical model is used, the relationship expressed in equation (1) means that the current at any of the electrodes at the four compass points can be "predicted" from the current values of the other three electrodes. Using the electrical model of equation (7) yields:

$$i_w^2 + i_e^2 = i_s^2 + i_n^2$$

Using the electrical model of equation (6) yields:

$$i_w + i_e = i_s + i_n$$

This provides a simple means to validate either electrical model.

Applying the tools of geometric and electrical modeling may help to create more efficient, accurate measurements of the nerve location.

While certain embodiments have been described, it will be appreciated by those skilled in the art that variations may be accomplished in view of these teachings without deviating from the spirit or scope of the present application. For example, the system 22 may be implemented using any combination of computer programming software, firmware or hardware. As a preparatory act to practicing the system 20 or constructing an apparatus according to the application, the computer programming code (whether software or firmware) according to the application will typically be stored in one or more machine readable storage mediums such as fixed (hard) drives, diskettes, optical disks, magnetic tape, semiconductor memories such as ROMs, PROMs, etc., thereby making an article of manufacture in accordance with the application. The article of manufacture containing the computer programming code may be used by either executing the code directly from the storage device, by copying the code from the storage device into another storage device such as a hard disk, RAM, etc. or by transmitting the code on a network for remote execution.

**Claims**

1. A system (20) comprising:
   an access system (34) for establishing an operative corridor to a spinal target site via a lateral, trans-psoas approach including a surgical accessory (30) having a plurality of stimulation electrodes (1402);
   a sensing electrode (27) configured to sense a neuromuscular response of a muscle coupled to a nerve depolarized by a stimulation current signal emitted from at least one of said plurality of stimulation electrodes (1402); wherein said sensing electrode includes an electromyographic (EMG) electrode and
   a control unit (22) capable of electrically stimulating said plurality of stimulation electrodes (1402) on said surgical accessory (30), receiving neuromuscular response data from said sensing electrode (28), and determining a direction from the surgical accessory to said nerve based upon the sensed response;
   wherein the control unit (22) is configured to:

   a) electrically stimulate a first stimulation electrode with a first current signal, determine whether a first stimulation current threshold at which the nerve will depolarize has been bracketed, stimulate a second stimulation electrode with a second current signal, determine whether a second stimulation current threshold at which the nerve will depolarize has been bracketed, wherein a stimulation current threshold at which the nerve will depolarize means a minimum stimulation current which produces a significant EMG response, and determining whether a stimulation current threshold has been bracketed means determining if a stimulation current threshold falls within an initial bracketing range;
   b) determine a first range for the first stimulation current threshold, and determine a second range for the second stimulation current threshold, each range having a maximum stimulation current threshold value and a minimum stimulation current threshold value;
   c) process the first and second ranges and display an arc indicating a general direction of a nerve from the surgical accessory;
   d) electrically stimulate the first stimulation electrode with a third current signal, determine whether the first stimulation current threshold has been bracketed, stimulate the second stimulation electrode with a fourth current signal and determine whether the second stimulation current threshold has been bracketed;
   e) electrically stimulate each electrode until a stimulation current threshold has been bracketed; and
   f) display an arc indicating a general direction of a nerve from the surgical accessory and narrow the arc as stimulation current thresholds are bracketed;
   g) electrically stimulate the first and second stimulation electrodes to bisect the first and second initial bracketing ranges until a first stimulation current threshold has been found for the first stimulation electrode and a second stimulation current threshold has been found for the second stimulation electrode within a predetermined range of accuracy; and

h) display an arc indicating a general direction of a nerve from the surgical accessory and narrow the arc as stimulation current threshold brackets are bisected.

2. The system of claim 1, wherein said access system (34) includes a series of sequential dilation cannulae, at least one cannula having said plurality of stimulation electrodes near a distal end.

3. The system (20) of claim 2, wherein said access system (34) further comprises a K-wire (46).

4. The system (20) of claim 3, wherein said K-wire (46) has a first stimulation electrode at a distal tip of the K-wire (46).

5. The system (20) of claim 4, wherein said K-wire (46) has a second stimulation electrode away from the distal tip.

6. The system (20) of claim 4, wherein said K-wire (46) is slidably received in the surgical accessory (30).

7. The system (20) of claim 1, further comprising a handle (52) coupled to the surgical accessory (30), the handle having at least one button for initiating the electrical stimulation from said control unit (22) to said plurality of stimulation electrodes on said surgical accessory.

8. The system (20) of claim 1, wherein the control unit (22) comprises a display (40) operable to display an electromyographic (EMG) response of the muscle.

9. The system (20) of claim 1, wherein the control unit (22) comprises a touch-screen display (40) operable to receive commands from a user.

10. The system (20) of claim 1, wherein said stimulation electrodes are positioned in a two-dimensional plane.

11. The system (20) of claim 1, wherein said stimulation electrodes are positioned orthogonally to form a cross.

12. The system (20) of claim 11, the control unit (22) derives x and y Cartesian coordinates of a nerve direction with respect to the surgical accessory by using $x = i_w^2 - i_e^2$ and $y = i_s^2 - i_n^2$ where $i_e$, $i_w$, $i_n$, and $i_s$ represent stimulation current thresholds for east, west, north, and south electrodes.

13. The system (20) of claim 1, wherein said plurality of stimulation electrodes comprises a first set of electrodes in a first two-dimensional plane and a second set of at least one electrode in another plane that is parallel to the first plane.

14. The system (20) of claim 13, wherein the stimulation electrodes form a tetrahedron.

15. The system (20) of claim 13, wherein the control unit (22) is configured to determine a three-dimensional vector from a reference point on the surgical accessory (30) to a nerve.

16. The system (20) of claim 15, wherein the control unit (22) is further configured to display the three-dimensional vector to a user.

17. The system (20) of claim 1, wherein said plurality of stimulation electrodes comprises two pairs of electrodes.

18. The system (20) of claim 1, wherein said plurality of stimulation electrodes comprises a first electrode at a first longitudinal level of the surgical accessory (30) and a second electrode at a second longitudinal level of the surgical accessory (30).

19. The system (20) of claim 1, wherein said first and second current signals are equal.

20. The system (20) of claim 1, wherein the control unit (22) is configured to emit a sound when the control unit (22) determines a distance between the surgical accessory (30) and the nerve has reached a predetermined level.

21. The system (20) of claim 1, wherein the control unit (22) is configured to emit a sound that indicates a distance between the surgical accessory (30) and the nerve.

**Patentansprüche**

1. System (20), umfassend:
   ein Zugangssystem (34) zum Erstellen eines operativen Gangs zu einer spinalen Zielstelle mittels eines lateralen Trans-Psoas-Ansatzes, umfassend ein chirurgisches Zubehör (30) mit einer Mehrzahl von Stimulationselektroden (1402);
   eine Messelektrode (27), welche dazu eingerichtet ist, eine neuromuskuläre Antwort eines Muskels zu messen, welcher mit einem Nerv gekoppelt ist, welcher durch ein Stimulationsstromsignal depolarisiert wird, welches von wenigstens einer der Mehrzahl von Stimulationselektroden (1402) ausgesendet worden ist;
   wobei die Messelektrode eine elektromyographische (EMG) Elektrode und eine Steuereinheit (22) umfasst, welche zum elektrischen Stimulieren der Mehrzahl von Stimulationselektroden (1402) an dem chirurgischen Zubehör (30), einem Empfangen von neuromuskulären Antwortdaten von der Messelektrode (28) und zum Bestimmen einer Richtung von dem chirurgischen Zubehör zu dem Nerv auf der Grundlage der gemessenen Antwort eingerichtet ist;
   wobei die Steuereinheit (22) dazu eingerichtet ist:

   a) eine erste Stimulationselektrode mit einem ersten Stromsignal elektrisch zu stimulieren, zu bestimmen, ob ein erster Stimulationsstrom-Schwellenwert, bei dem der Nerv depolarisiert wird, eingeklammert worden ist, eine zweite Stimulationselektrode mit einem zweiten Stromsignal zu stimulieren, zu bestimmen, ob ein zweiter Stimulationsstrom-Schwellenwert, bei dem der Nerv depolarisiert wird, eingeklammert worden ist, wobei ein Stimulationsstrom-Schwellenwert, bei welchem der Nerv depolarisiert wird, einen minimalen Stimulationsstrom bezeichnet, welcher eine signifikante EMG-Antwort erzeugt, und wobei das Bestimmen, ob ein Stimulationsstrom-Schwellenwert eingeklammert worden ist, ein Bestimmen bezeichnet, ob ein Stimulationsstrom-Schwellenwert in einen initialen Klammerbereich fällt;
   b) einen ersten Bereich für den ersten Stimulationsstrom-Schwellenwert zu bestimmen und einen zweiten Bereich für den zweiten Stimulationsstrom-Schwellenwert zu bestimmen, wobei jeder Bereich einen maximalen Stimulationsstrom-Schwellenwert und einen minimalen Stimulationsstrom-Schwellenwert aufweist;
   c) den ersten und den zweiten Bereich zu verarbeiten und einen Bogen darzustellen, welcher eine wesentliche Richtung eines Nervs von dem chirurgischen Zubehör anzeigt;
   d) die erste Stimulationselektrode mit einem dritten Stromsignal elektrisch zu stimulieren, zu bestimmen, ob der erste Stimulationsstrom-Schwellenwert eingeklammert worden ist, die zweite Stimulationselektrode mit einem vierten Stromsignal zu stimulieren, und zu bestimmen, ob der zweite Stimulationsstrom-Schwellenwert eingeklammert worden ist;
   e) jede Elektrode elektrisch zu stimulieren bis ein Stimulationsstrom-Schwellenwert eingeklammert worden ist, und
   f) einen Bogen darzustellen, welcher eine wesentliche Richtung eines Nervs von dem chirurgischen Zubehör anzeigt und den Bogen einzuschränken, wenn Stimulationsstrom-Schwellenwerte eingeklammert sind;
   g) die erste und die zweite Stimulationselektrode elektrisch zu stimulieren, um den ersten und den zweiten initialen Einklammerungsbereich zu halbieren bis innerhalb eines vorbestimmten Genauigkeitsbereichs ein erster Stimulationsstrom-Schwellenwert für die erste Stimulationselektrode gefunden worden ist und ein zweiter Stimulationsstrom-Schwellenwert für die zweite Stimulationselektrode gefunden worden ist; und
   h) einen Bogen darzustellen, welcher eine wesentliche Richtung eines Nervs von dem chirurgischen Zubehör anzeigt, und den Bogen einzuschränken, wenn Stimulationsstrom-Schwellenwert-Klammern halbiert sind.

2. System nach Anspruch 1, wobei das Zugangssystem (34) eine Reihe von sequenziellen Erweiterungskanülen umfasst, wobei wenigstens eine Kanüle die Mehrzahl von Stimulationselektroden nahe einem distalen Ende aufweist.

3. System (20) nach Anspruch 2, wobei das Zugangssystem (34) ferner einen K-Draht (46) umfasst.

4. System (20) nach Anspruch 3, wobei der K-Draht (46) eine erste Stimulationselektrode an einer distalen Spitze des K-Drahtes (46) aufweist.

5. System (20) nach Anspruch 4, wobei der K-Draht (46) eine von der distalen Spitze entfernte zweite Stimulationselektrode aufweist.

6. System (20) nach Anspruch 4, wobei der K-Draht (46) in dem chirurgischen Zubehör (30) verschiebbar aufgenommen ist.

7. System (20) nach Anspruch 1, ferner umfassend einen Griff (52), welcher mit dem chirurgischen Zubehör (30) gekoppelt ist, wobei der Griff wenigstens einen Knopf zum Einleiten der elektrischen Stimulation von der Steuereinheit (22) zu der Mehrzahl von Stimulationselektroden an dem chirurgischen Zubehör aufweist.

8. System (20) nach Anspruch 1, wobei die Steuereinheit (22) eine Anzeigeeinheit (40) umfasst, welche betriebsfähig ist, um eine elektromyographische (EMG) Antwort des Muskels anzuzeigen.

9. System (20) nach Anspruch 1, wobei die Steuereinheit (22) eine Touch-Screen-Anzeigeeinheit (40) umfasst, welche betriebsfähig ist, um Befehle von einem Bediener zu empfangen.

10. System (20) nach Anspruch 1, wobei die Stimulationselektroden in einer zweidimensionalen Ebene positioniert sind.

11. System (20) nach Anspruch 1, wobei die Stimulationselektroden orthogonal positioniert sind, um ein Kreuz auszubilden.

12. System (20) nach Anspruch 11, wobei die Steuereinheit (22) kartesische x- und y-Koordinaten einer Nervenrichtung in Bezug auf das chirurgische Zubehör unter Verwendung von $x = i_w^2 - i_e^2$ und $y = i_s^2 - i_n^2$ ableitet, wobei $i_e$, $i_w$, $i_n$ und $i_s$ Stimulationsstrom-Schwellenwerte für eine östliche, eine westliche, eine nördliche und eine südliche Elektrode darstellen.

13. System (20) nach Anspruch 1, wobei die Mehrzahl von Stimulationselektroden einen ersten Satz von Elektroden in einer ersten zweidimensionalen Ebene und einen zweiten Satz von wenigstens einer Elektrode in einer anderen Ebene umfasst, welche parallel zu der ersten Ebene verläuft.

14. System (20) nach Anspruch 13, wobei die Stimulationselektroden einen Tetraeder ausbilden.

15. System (20) nach Anspruch 13, wobei die Steuereinheit (22) dazu eingerichtet ist, einen dreidimensionalen Vektor von einem Referenzpunkt an dem chirurgischen Zubehör (30) zu einem Nerv zu bestimmen.

16. System (20) nach Anspruch 15, wobei die Steuereinheit (22) ferner dazu eingerichtet ist, einem Benutzer den dreidimensionalen Vektor anzuzeigen.

17. System (20) nach Anspruch 1, wobei die Mehrzahl von Stimulationselektroden zwei Paare von Elektroden umfasst.

18. System (20) nach Anspruch 1, wobei die Mehrzahl von Stimulationselektroden eine erste Elektrode bei einem ersten longitudinalen Niveau des chirurgischen Zubehörs (30) und eine zweite Elektrode bei einem zweiten longitudinalen Niveau des chirurgischen Zubehörs (30) umfasst.

19. System (20) nach Anspruch 1, wobei das erste und das zweite Stromsignal gleich sind.

20. System (20) nach Anspruch 1, wobei die Steuereinheit (22) dazu eingerichtet ist, ein Geräusch auszusenden, wenn die Steuereinheit (22) bestimmt, dass ein Abstand zwischen dem chirurgischen Zubehör (30) und dem Nerv ein vorbestimmtes Niveau erreicht hat.

21. System (20) nach Anspruch 1, wobei die Steuereinheit (22) dazu eingerichtet ist, ein Geräusch auszusenden, welches einen Abstand zwischen dem chirurgischen Zubehör (30) und dem Nerv anzeigt.

**Revendications**

1. Système (20) comprenant :

un système d'accès (34) pour établir un couloir opérationnel vers un site cible vertébral via une approche transpsoas transversale comprenant un accessoire chirurgical (30) ayant une pluralité d'électrodes de stimulation (1402) ;
une électrode de détection (27) configurée pour détecter une réponse neuromusculaire d'un muscle couplé à un nerf dépolarisé par un signal de courant de stimulation émis par au moins l'une de ladite pluralité d'électrodes de stimulation (1402) ; ladite électrode de détection comprenant une électrode électromyographique (EMG), et une unité de commande (22) capable de stimuler électriquement ladite pluralité d'électrodes de stimulation (1402) sur ledit accessoire chirurgical (30), recevoir des données de réponse neuromusculaire de ladite électrode de détection (28), et déterminer une direction, de l'accessoire chirurgical audit nerf, en fonction de la réponse détectée ;

dans lequel l'unité de commande (22) est configurée pour :

a) stimuler électriquement une première électrode de stimulation avec un premier signal de courant, déterminer si un premier seuil de courant de stimulation auquel le nerf se dépolarise, est inclus dans un encadrement, stimuler une seconde électrode de stimulation avec un deuxième signal de courant, déterminer si un second seuil de courant de stimulation auquel le nerf se dépolarise, est inclus dans un encadrement, où un seuil de courant de stimulation auquel le nerf se dépolarise signifie un courant de stimulation minimum qui produit une réponse EMG significative, et déterminer si un seuil de courant de stimulation est inclus dans un encadrement signifie déterminer si un seuil de courant de stimulation se trouve dans une plage d'encadrement initiale ;
b) déterminer une première plage pour le premier seuil de courant de stimulation, et déterminer une seconde plage pour le second seuil de courant de stimulation, chaque plage ayant une valeur de seuil de courant de stimulation maximum et une valeur de seuil de courant de stimulation minimum ;
c) traiter les première et seconde plages et afficher un arc indiquant une direction générale d'un nerf à partir de l'accessoire chirurgical ;
d) stimuler électriquement la première électrode de stimulation avec un troisième signal de courant, déterminer si le premier seuil de courant de stimulation est inclus dans un encadrement, stimuler la seconde électrode de stimulation avec un quatrième signal de courant et déterminer si le second seuil de courant de stimulation est inclus dans un encadrement ;
e) stimuler électriquement chaque électrode jusqu'à ce qu'un seuil de courant de stimulation ait été inclus dans un encadrement ; et
f) afficher un arc indiquant une direction générale d'un nerf à partir de l'accessoire chirurgical et rétrécir l'arc lorsque les seuils de courant de stimulation sont inclus dans un encadrement ;
g) stimuler électriquement les première et seconde électrodes de stimulation pour couper les première et seconde plages d'encadrement initiales jusqu'à ce qu'un premier seuil de courant de stimulation ait été trouvé pour la première électrode de stimulation et qu'un second seuil de courant de stimulation ait été trouvé pour la seconde électrode de stimulation dans une plage de précision prédéterminée ; et
h) afficher un arc indiquant une direction générale d'un nerf à partir de l'accessoire chirurgical et rétrécir l'arc lorsque les encadrements de seuil de courant de stimulation sont coupés.

2. Système selon la revendication 1, dans lequel ledit système d'accès (34) comprend une série de canules de dilatation séquentielles, au moins une canule ayant ladite pluralité d'électrodes de stimulation à proximité d'une extrémité distale.

3. Système (20) selon la revendication 2, dans lequel ledit système d'accès (34) comprend en outre une broche de Kirschner (46).

4. Système (20) selon la revendication 3, dans lequel ladite broche de Kirschner (46) a une première électrode de stimulation au niveau de la pointe distale de la broche de Kirschner (46).

5. Système (20) selon la revendication 4, dans lequel ladite broche de Kirschner (46) a une seconde électrode de stimulation à distance de la pointe distale.

6. Système (20) selon la revendication 4, dans lequel ladite broche de Kirschner (46) est reçue de manière coulissante dans l'accessoire chirurgical (30).

7. Système (20) selon la revendication 1, comprenant en outre une poignée (52) couplée à l'accessoire chirurgical (30), la poignée ayant au moins un bouton pour initier la stimulation électrique à partir de ladite unité de commande (22) à ladite pluralité d'électrodes de stimulation sur ledit accessoire chirurgical.

8. Système (20) selon la revendication 1, dans lequel l'unité de commande (22) comprend un affichage (40) ayant pour fonction d'afficher une réponse électromyographique (EMG) du muscle.

**9.** Système (20) selon la revendication 1, dans lequel l'unité de commande (22) comprend un affichage à écran tactile (40) ayant pour fonction de recevoir des commandes d'un utilisateur.

**10.** Système (20) selon la revendication 1, dans lequel lesdites électrodes de stimulation sont positionnées dans un plan bidimensionnel.

**11.** Système (20) selon la revendication 1, dans lequel lesdites électrodes de stimulation sont positionnées de manière orthogonale afin de former une croix.

**12.** Système (20) selon la revendication 11, l'unité de commande (22) dérive les coordonnées cartésiennes x et y d'une direction de nerf par rapport à l'accessoire chirurgical en utilisant $x = i_w^2 - i_e^2$ et $y = i_s^2 - i_n^2$ où $i_s$, $i_w$, $i_n$, et $i_s$ représentent des seuils de courant de stimulation pour les électrodes est, ouest, nord et sud.

**13.** Système (20) selon la revendication 1, dans lequel ladite pluralité d'électrodes de stimulation comprend un premier ensemble d'électrodes dans un premier plan bidimensionnel et un second ensemble d'au moins une électrode dans un autre plan qui est parallèle au premier plan.

**14.** Système (20) selon la revendication 13, dans lequel les électrodes de stimulation forment un tétraèdre.

**15.** Système (20) selon la revendication 13, dans lequel l'unité de commande (22) est configurée pour déterminer un vecteur tridimensionnel à partir d'un point de référence sur l'accessoire chirurgical (30) jusqu'à un nerf.

**16.** Système (20) selon la revendication 15, dans lequel l'unité de commande (22) est en outre configurée pour afficher le vecteur tridimensionnel à un utilisateur.

**17.** Système (20) selon la revendication 1, dans lequel ladite pluralité d'électrodes de stimulation comprend deux paires d'électrodes.

**18.** Système (20) selon la revendication 1, dans lequel ladite pluralité d'électrodes de stimulation comprend une première électrode à un premier niveau longitudinal de l'accessoire chirurgical (30) et une seconde électrode à un second niveau longitudinal de l'accessoire chirurgical (30) .

**19.** Système (20) selon la revendication 1, dans lequel lesdits premier et second signaux de courant sont identiques.

**20.** Système (20) selon la revendication 1, dans lequel l'unité de commande (22) est configurée pour émettre un son lorsque l'unité de commande (22) détermine qu'une distance entre l'accessoire chirurgical (30) et le nerf a atteint un niveau prédéterminé.

**21.** Système (20) selon la revendication 1, dans lequel l'unité de commande (22) est configurée pour émettre un son qui indique une distance entre l'accessoire chirurgical (30) et le nerf.

Providing four orthogonally-disposed electrodes around the periphery of a surgical access instrument

Stimulating the electrodes to identify the current threshold ($I_{Thresh}$) for the muscle myotome associated with the nerve adjacent to the surgical access instrument

Determining the direction of the surgical access instrument relative to the nerve via successive approximation

Communicating the successive approximation direction information to the surgeon in an easy-to-interpret fashion

FIG. 1

**"SEQUENTIAL DILATION ACCESS SYSTEM"**

FIG. 2

PATIENT

NERVES

MUSCLE GROUPS

46-50, 56, 62

52, 54

SURGICAL ACCESSORY

SURGICAL ACCESSORY HANDLE

Stimulation Anode

28

26

EMG ELECTRODES

EMG Common

20

24

PATIENT MODULE

20

22

CONTROL UNIT

AUDIO

18

23

EP 1 594 401 B1

FIG. 3

Stim
Current

200μs

$I_{STIM}$

Time

FIG. 4

EMG
Voltage

$V_{max}$

$V_{pp}$

$V_{min}$

Time

FIG. 5

FIG. 6

EP 1 594 401 B1

FIG. 7A

Vpp

Stimulation Threshold
to be found

Stimulation mA

FIG. 7B

2    4         8                    16

First Bracket

EMG Response – NO          EMG Response – YES
Lower Boundary                 Upper Boundary

FIG. 7C

2    4         8        12        16
                   1/2         1/2

Second Bracket

EMG Response – NO          EMG Response – YES
Lower Boundary                 Upper Boundary

this step repeated until bracket size is    0.2 mA

EP 1 594 401 B1

Vpp

Stimulation Threshold
to be found

Stimulation mA

FIG. 7A

FIG. 7D

0.1 mA Increments

result

2  4  8  12  16

FIG. 7E

EMG Response – NO
Lower Boundary

EMG Response – YES
Upper Boundary

Switching between response and no response

2  4  8  12  16

FIG. 8

FIG. 9

FIG. 10

FIG. 11

stimulus pulse

$V_{max}$

$V_{min}$

$t_1$

$t_2$

FIG. 12

FIG. 13

1402A

1400

1402B

1402C

1404

FIG. 14A

1402 A

1402D

1402B

1402C

FIG. 14B

33

FIG. 15A

FIG. 15B

FIG. 15C

FIG. 16

FIG. 17

EP 1 594 401 B1

FIG. 18

FIG. 19

FIG. 20

EP 1 594 401 B1

FIG. 21

41

2202

2200 A

$d_1$

$d_2$

D    D

2200 B

d

FIG. 22

y
north

west    R    east    x

south

z    2302

D

D

x

k-wire

2300

FIG. 23

FIG. 24

FIG. 25

FIG. 26

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2003026482 A

- US 2012238893 A